# EUROPEAN PATENT SPECIFICATION

(11) **EP 4 665 839 B1**
(45) Date of publication and mention of the grant of the patent: **25.11.2026**
(21) Application number: 24704812.7
(22) Date of filing: 14.02.2024
(51) Int. Cl.: C12N 5/00, C12N 5/09

(54) **CELL CULTURE MEDIA**
ZELLKULTURMEDIUM
MILIEU DE CULTURE CELLULAIRE

(30) Priority: 16.02.2023 EP 23156964
(43) Date of publication of application: 24.12.2025
(73) Proprietor: Merck Patent GmbH, 64293 Darmstadt (DE)
(72) Inventor: ZIMMER, Aline, 64293 Darmstadt (DE); MERKEL, Corinna, 64293 Darmstadt (DE); MUELLER, Mareike, 20099 Hamburg (DE)
(74) Representative: Merck Patent Association
(86) International application number: PCT/EP2024/053727
(87) International publication number: WO 2024/170622

(56) References cited:
- WO-A1-2012/019160
- US-A1- 2017 058 256
- NATASCHA VERHAGEN: "-tyrosine in CHO cells", ENGINEERING IN LIFE SCIENCES, vol. 20, no. 9-10, 8 June 2020 (2020-06-08), DE, pages 384 - 394, XP093166771, ISSN: 1618-0240, Retrieved from the Internet <URL:https://onlinelibrary.wiley.com/doi/full-xml/10.1002/elsc.202000017> DOI: 10.1002/elsc.202000017
- BIELSER JEAN-MARC ET AL: "Perfusion mammalian cell culture for recombinant protein manufacturing - A critical review", BIOTECHNOLOGY ADVANCES, ELSEVIER PUBLISHING, BARKING, GB, vol. 36, no. 4, 5 May 2018 (2018-05-05), pages 1328 - 1340, XP085400132, ISSN: 0734-9750, DOI: 10.1016/J.BIOTECHADV.2018.04.011

## Description

The present invention relates to cell culture media comprising N-norvalyl-L-tyrosine. The poor solubility of tyrosine in cell culture media is overcome by substituting it partially or fully with N-norvalyl-L-tyrosine.

Cell culture media support and maintain the growth of cells in an artificial environment.

Depending on the type of organism whose growth shall be supported, the cell culture media comprise a complex mixture of components, sometimes more than one hundred different components.

The cell culture media required for the propagation of mammalian, insect or plant cells are typically much more complex than the media to support the growth of bacteria and yeasts.

The first cell culture media that were developed consisted of undefined components, such as plasma, serum, embryo extracts, or other non-defined biological extracts or peptones. A major advance was made with the development of chemically defined media. Chemically defined media often comprise but are not exclusively limited to amino acids, vitamins, metal salts, antioxidants, chelators, growth factors, buffers, hormones, and many more substances known to those experts in the art.

Some cell culture media are offered as sterile aqueous liquids. The disadvantage of liquid cell culture media is their reduced shelf life and difficulties for shipping and storage. As a consequence, many cell culture media are presently offered as finely milled dry powder mixtures. They are manufactured for the purpose of dissolving in water and/or aqueous solutions and in the dissolved state are designed, often with other supplements, for supplying cells with a substantial nutrient base for growth and/or production of biopharmaceuticals from said cells.

Many biopharmaceutical production platforms are based on fed-batch cell culture protocols. The aim typically is to develop high-titer cell culture processes to meet increasing market demands and reduce manufacturing costs. Besides fed-batch, perfusion is also a common cultivation method for biopharmaceutical production in N stage bioreactor but also regarding N-1 bioreactor. The aim typically is to reduce seed train time by reaching higher cell density, as well as higher volumetric productivity while maintaining a high viability.

Beside the use of high-performing recombinant cell lines, improvements in cell culture media and process parameters are required to realize the maximum production potentials.

In a fed-batch process, a basal medium supports initial growth and production, and a feed medium prevents depletion of nutrients and sustains the production phase. The media are chosen to accommodate the distinct metabolic requirements during different production phases. Process parameter settings - including feeding strategy and control parameters - define the chemical and physical environments suitable for cell growth and protein production.

In a perfusion process, a basal medium supports initial growth and production to a specific point (still exponential growth phase, no limitation in nutrients), where the medium is being simultaneously added and removed from the bioreactor at a specific media exchange rate, which can be described either by the perfusion rate (P, d⁻¹) in volume of medium per bioreactor working volume per day (VVD, d⁻¹) or by the cell specific perfusion rate (CSPR, pL/cell/d). CSPR displays the nutrient supply per cell and day in a continuous process. Hence, CSPR is cell line and medium specific.

Optimization of the feed medium is a major aspect in the optimization of a fed-batch process. Mostly the feed medium is highly concentrated to avoid dilution of the product (antibody or recombinant proteins) in the bioreactor. The controlled addition of the nutrient directly affects the growth rate and the longevity of the culture.

Regarding continuous processes, optimization of the perfusion medium as well as minimizing CSPR are major aspects. It is well known from literature, that concentrated media can be used to reduce CSPR.

Amino acids (AA) like tyrosine are essential components of cell culture media since they are key to support cellular growth. In addition, AA are key building blocks for recombinant proteins produced using mammalian cell culture technologies. The solubility of tyrosine is a limiting factor hindering the concentration of cell culture media (CCM) and feed formulations. Such a concentration would be essential to develop next generation manufacturing platforms. Particularly, highly concentrated formulations are required for biomanufacturing processes using inline dilution, to reduce the volume of CCM which must be stored in tanks (= reduce manufacturing footprint) or in general to a. reduce the volume of feed added throughout a fed-batch (FB) process or b. to reduce the CSPR by concentrating media in continuous processes and thus potentially increase the volumetric titer.

Consequently it would be favourable to find a way to improve the solubility of tyrosine.

Another key characteristic for amino acid derivatives, in particular when used in media, is that they need to be readily available to cells, to support metabolic requirements. Many amino acid derivatives are not readily bioavailable and require the release of enzymes to cleave them thus releasing the bioavailable canonical amino acid. Consequently, many highly soluble amino acid derivatives may not be suitable for cell culture media.

US 2019/0390161 discloses a method of culturing cells using a cell culture medium, comprising at least one oligopeptide of 2-10 amino acids, whereby those amino acids are natural and selected from the following amino acids: Cys, Cyss, Leu, Tyr, Val, Ile.

WO 2012/019160 discloses a method of culturing CHO cells with a serum-free defined production medium and feed supplemented with certain dipeptides including the Tyr-containing dipeptides Tyr-His, Tyr-Lys, Tyr-Ala, and Tyr-Val.

It has been unexpectedly found that tyrosine can be replaced in cell culture media by N-norvalyl-L-tyrosine or salts thereof. In addition to its use as tyrosine source, the N-norvalyl derivative presents a higher solubility compared to tyrosine and can therefore be used in highly concentrated formulations. In addition, compared to other tyrosine derivatives it is readily available to cells thus ensuring the continuous tyrosine supply required for growth and recombinant protein production.

The present invention is therefore directed to cell culture media comprising N-norvalyl-L-tyrosine. If in the following this component is mentioned, it means the free dipeptide as well as salts thereof like the hydrochloride, Na⁺, K⁺, Mg²⁺, Ca²⁺, Li⁺, preferably the hydrochloride thereof. A person skilled in the art is aware that either the free dipeptide can be used or any salt thereof.

In a preferred embodiment, the cell culture medium comprises the hydrochloride salt of N-norvalyl-L-tyrosine.

In a preferred embodiment, the cell culture medium is a dry powder medium.

In one embodiment, especially if the cell culture medium is a basal medium or a perfusion cell culture medium, it comprises N-norvalyl-L-tyrosine as well as optionally native tyrosine. Native in this case means the unmodified amino acid and/or a salt thereof.

In another preferred embodiment, the cell culture medium is a feed medium.

The feed medium may comprise N-norvalyl-L-tyrosine and the corresponding native tyrosine but it may also only comprise N-norvalyl-L-tyrosine and not the corresponding native tyrosine.

In a preferred embodiment the cell culture medium comprises N-norvalyl-L-tyrosine but no tyrosine. No tyrosine means no L-tyrosine and/or salts thereof at all or L-tyrosine and/or salts thereof in a molar ratio of less than 1/10 (L-tyrosine and/or salts thereof / N-norvalyl-L-tyrosine).

In another preferred embodiment the cell culture medium is a liquid medium having a pH of 8.5 or less and comprising at least N-norvalyl-L-tyrosine in a concentration above 1 mmol/l. In case of feed media the concentration is typically above 10 mmol/l. The upper limit is only defined by the solubility of N-norvalyl-L-tyrosine. The solubility may depend on the solvent, the pH and the salt concentration. Consequently, it is typically possible to generate liquid media with a concentration of N-norvalyl-L-tyrosine of up to 100 mmol/L.

In a preferred embodiment the pH of the liquid medium is between 6.0 and 8.5, most preferred between 6.5 and 7.8.

In one embodiment the medium is in in x-fold concentrated form relative to the concentration of said medium in use, whereby x is between 1.5 and 100, e.g. 2-fold, 3-fold, 4-fold, 5-fold or 10-fold concentrated form.

In one embodiment, the cell culture medium comprises at least one or more saccharide components, one or more amino acids, one or more vitamins or vitamin precursors, one or more salts, one or more buffer components, one or more co-factors and one or more nucleic acid components.

The present invention is further directed to a method for producing a cell culture medium according to the present invention by
a) mixing N-norvalyl-L-tyrosine with the other components of the cell culture medium. In this case components are in dry state.
b) subjecting the mixture of step a) to milling

In a preferred embodiment step b) is performed in a pin mill, fitz mill or a jet mill.

In another preferred embodiment, the mixture from step a) is cooled to a temperature below 0°C prior to milling.

The present invention is further directed to a process for culturing cells by
a) providing a bioreactor
b) optionally generating a liquid cell culture medium according to the present invention by dissolving a dry powder medium according to the present invention in an aqueous liquid. This step is required in case the cell culture medium comprising N-norvalyl-L-tyrosine is a dry powder medium, it is not required if the medium is already present in form of a liquid medium.
c) mixing in said bioreactor the cells to be cultured with a liquid cell culture medium according to the present invention.
d) incubating the mixture of step b).

In one embodiment, the bioreactor is a perfusion bioreactor.

In a preferred embodiment the process for culturing cells is a process for perfusion cell culture, comprising culturing cells in a perfusion bioreactor with a media inlet and a harvest outlet whereby
i. continuously or one or several times during the cell culture process new cell culture medium according to the present invention is inserted into the bioreactor via the media inlet
ii. continuously or one or several times during the cell culture process harvest is removed from the bioreactor via the harvest outlet

Preferably, during perfusion phase, new cell culture medium comprising N-norvalyl-L-tyrosine is continuously inserted into the bioreactor via the media inlet and harvest is continuously removed from the bioreactor.

The present invention is also directed to a fed batch process for culturing cells in a bioreactor by
- Filling into a bioreactor cells and an aqueous cell culture medium
- Incubating the cells in the bioreactor
- Adding a cell culture medium, which is in this case a feed medium, to the bioreactor, continuously over the whole time or once or several times within the cells incubation time
whereby the feed medium is a liquid cell culture medium according to the present invention comprising at least N-norvalyl-L-tyrosine.

Preferably the feed medium has a pH below pH 8.5 and comprises at least N-norvalyl-L-tyrosine in a concentration above 10 mmol/L.
Figure 1 shows the maximal solubility of tyrosine and the norvalyl-L-tyrosine derivative in MQ-water at 25°C and pH 7.3. Further details can be found in Example 1.
Figure 2 shows the solubility of Tyr 2Na salt 2H₂O and its respective norvalyl-L-tyrosine derivative or salts thereof in 6-fold concentrated CCM (pH 7.3±0.1) at 25°C. Solubility was assessed by dissolving increasing amount of Tyrosine derivative in 6-fold concentrated media and turbidity was measured. A solution having a turbidity below 5 NTU is considered soluble. Further details can be found in Example 2.
Figure 3 shows the determination of the maximum solubility of Tyr 2Na salt 2H₂O and *N*-L-NVa-L-Tyr HCl in 6-fold concentrated modified EX-CELL^{®} Advanced HD Perfusion Medium formulation depleted in Tyr. Further details can be found in Example 3.
Figure 4 shows the stability of 6-fold concentrated CCM containing Tyr 2Na salt 2H₂O or *N*-L-NVa-Tyr HCl analyzed by UPLC. Further details can be found in Example 4.
Figure 5 shows the cell performance of CHOK1 GS clone A in batch process. Further details can be found in Example 5.
Figure 6 shows the cell performance of CHOK1 non-GS clone in batch process. Further details can be found in Example 6.
Figure 7 shows the cell performance of CHOZN GS clone A in batch process. Further details can be found in Example 7.
Figure 8 shows the cell performance of CHOZN GS clone B in batch culture. Further details can be found in Example 8.
Figure 9 shows the cell performance of CHOZN GS clone C in batch culture. Further details can be found in Example 9.
Figure 10 shows the cell performance of CHOZN GS clone D in batch culture. Further details can be found in Example 10.
Figure 11 shows the cell performance of CHOZN GS clone E in batch culture. Further details can be found in Example 11.
Figures 12, 13 and 14 show features of recombinant proteins. Further details can be found in Example 12.
Figures 15, 16, 17 and 18 show the cell performance of CHOZN GS clone D, CHOZN GS clone F, CHO DG44 and CHOZN GS clone G in dynamic perfusion culture. Further details can be found in Example 13.

N-norvalyl-L-tyrosine is a dipeptide in which the non-proteinogenic amino acid norvaline is covalently attached to tyrosine via a peptide bond.

N-norvalyl-L-tyrosine is a product e.g. obtainable by chemical or biological, e.g. enzymatic, synthesis. Typically it is produced by chemical synthesis, e.g. according to known methods for dipeptide synthesis.

An exemplary synthesis of D-/L-norvalyl-L-tyrosine is shown in Abderhalden, E., & Bahn, A. (1930). Vergleichende Studien an Hand homologer Dipeptide des I-Tyrosins und der zugehörigen Halogenacylkörper über den Einfluβ ihrer Konstitution auf ihre Spaltbarkeit durch Alkali, Erepsin und Trypsin-Kinase. Fermentforschung, 11, 224 by
a) Synthesis of DL-α-bromo-n-valeryl-L-tyrosine ester via reaction of L-tyrosine ethyl ester with DL-α-bromo-n-valeric acid chloride
b) Hydrolysis of DL-α-bromo-n-valeryl-L-tyrosine ester, followed by evaporation
c) Amination of DL-α-bromo-n-valeryl-L-tyrosine with ammonia, followed by evaporation
d) Purification

N-norvalyl-L-tyrosine can be either N-L-norvalyl-L-tyrosine or N-D-norvalyl-L-tyrosine or mixtures thereof. For most cells the choice of either N-L-norvalyl-L-tyrosine or N-D-norvalyl-L-tyrosine or mixtures thereof does not make a difference. But in case of unwanted effects of one of the isomers, the skilled person can easily choose to prepare the cell culture media to be used according to the present invention with only one isomer.

As an example N-L-norvalyl-L-tyrosine, also called (2S)-2-[[(2S)-2-aminopentanoyl]amino]-3-(4-hydroxyphenyl)propanoic acid, is shown in formula I.

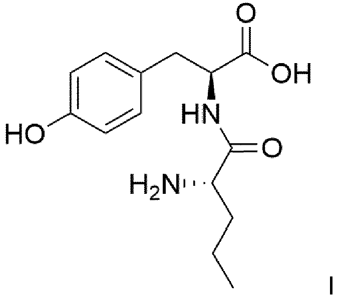

N-norvalyl-L-tyrosine according to the present invention also means any salts thereof like e.g. Na⁺, K⁺, Mg²⁺, Ca²⁺, Li⁺, preferably Na⁺, most preferred the HCl salt thereof as shown in formula II for N-L-norvalyl-L-tyrosine:

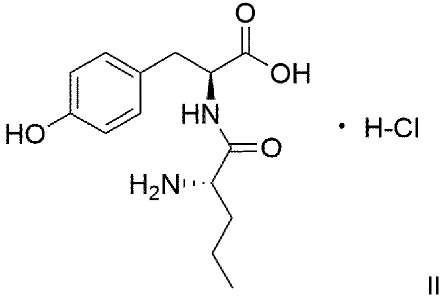

A cell culture medium according to the present invention is any mixture of components which maintains and/or supports the *in vitro* growth of cells like mammalian, insect or plant cells as well as bacteria and yeasts. It might be a complex medium or a chemically defined medium. The cell culture medium can comprise all components necessary to maintain and/or support the *in vitro* growth of cells or only some components so that further components are added separately. Examples of cell culture media according to the present invention are full media which comprise all components necessary to maintain and/or support the *in vitro* growth of cells as well as media supplements or feeds. In a preferred embodiment, the cell culture medium is a full medium or a feed medium. A full medium also called basal medium typically has a pH between 6.5 and 7.8. A feed medium preferably has a pH below 8.5, preferably between 6.0 and 8.5. A basal medium can be a medium used for batch, fed batch or perfusion cell culture.

Typically, the cell culture media according to the invention are used to maintain and/or support the growth of cells in a bioreactor.

A feed or feed medium is a cell culture medium which is not the basal medium that supports initial growth and production in a cell culture but the medium which is added at a later stage to prevent depletion of nutrients and sustains the production phase. A feed medium can have higher concentrations of some components compared to a basal culture medium. For example, some components, such as, for example, nutrients including amino acids or carbohydrates, may be present in the feed medium at about 5X, 6X, 7X, 8X, 9X, 10X, 12X, 14X, 16X, 20X, 30X, 50X, 100x, 200X, 400X, 600X, 800X, or even about 1000X of the concentrations in a basal medium.

But also other media beside feed media may be present and used in concentrated form.

A concentrated perfusion medium for example is a cell culture medium which supports growth and production after initial growth and production phase, once media exchange and thus perfusion is initiated. Concentrated perfusion media can be used to reduce CSPR and thus to increase volumetric productivity. A concentrated perfusion medium can have higher concentrations compared to a basal culture medium. Concentrated perfusion medium may have >1X to even about 100x of the concentration of a basal culture medium.

Media according to the present invention can thus have the concentration equivalent to the concentration in use which is the concentration of the medium in the bioreactor in contact with the cells. The media can also be in concentrated form. Such concentrates can be used for storage and can be diluted prior to addition to the bioreactor either off line or by in-line dilution. The concentrated media can also be added to the bioreactor as concentrates without further dilution. This is often done in case of concentrated feed media which are used in a fed batch process or in case of concentrated perfusion media which are continuously or once or several times added to the perfusion bioreactor during perfusion phase.

The culture medium of the present invention can thus be in concentrated form. It may be, e.g., in 2-fold, 3-fold, 4-fold, 5-fold, 10-fold, 20-fold, 50-fold or 100-fold concentrated form (relative to a concentration in use that supports growth and product formation of the cells in the bioreactor in contact with the cells).

If such concentrated culture media are used for preparing the culture medium for use, the concentrated culture medium is diluted with an aqueous solvent, preferably sterile water.

The concentrated media according to the present invention can be liquid media or dry powder media, whereby the dry powder media are provided with an instruction about which amount of aqueous solvent is to be used for dissolution of the dry powder medium to generate the concentrated liquid medium. Concentrations of media ingredients provided herein are always directed to the concentration in the respective liquid medium whereby the skilled person is aware that dry powder media are dissolved in a certain amount of aqueous solvent to give the respective liquid medium with a certain concentration of ingredients.

A mammalian cell culture medium is a mixture of components which maintain and/or support the *in vitro* growth of mammalian cells. Examples of mammalian cells are human or animal cells, preferably CHO cells, COS cells, I VERO cells, BHK cells, AK-1 cells, SP2/0 cells, L5.1 cells, hybridoma cells or human cells.

Chemically defined cell culture media are cell culture media that do not comprise any chemically undefined substances. This means that the chemical composition of all the chemicals used in the media is known. The chemically defined media do not comprise any yeast, animal or plant tissues; they do not comprise feeder cells, serum, extracts or digests or other components which may contribute to chemically poorly defined proteins in the media. Chemically undefined or poorly defined chemical components are those whose chemical composition and structure is not known, are present in varying composition or could only be defined with enormous experimental effort - comparable to the evaluation of the chemical composition and structure of a protein like albumin or casein.

A powdered cell culture medium or a dry powder medium is a cell culture medium typically resulting from a milling process, a lyophilisation process or a dry or wet granulation process. That means the powdered cell culture medium is a granular, particulate medium - not a liquid medium. The term "dry powder" may be used interchangeably with the term "powder;" however, "dry powder" as used herein simply refers to the gross appearance of the granulated material and is not intended to mean that the material is completely free of complexed or agglomerated solvent unless otherwise indicated. Dry powder media resulting from a milling or lyophilisation process typically have particle sizes below 0.5 mm, e.g. between 0.05 and 0.5 mm.

Dry powder media resulting from dry or wet granulation process, e.g. by spray drying, wet granulation or dry compaction, typically have particle sizes above 0.5 mm, e.g. between 0.5 and 5 mm. Dry compaction is typically done in a roll press. US 6,383,810 B2 discloses a method of producing an agglomerated eukaryotic cell culture medium powder. The method comprises wetting a dry powder cell culture medium with a solvent and then re-drying the moistened medium to obtain a dry agglomerated cell culture medium.

In one embodiment the dry powder media according to the present invention are produced by dry compaction.

Cells to be cultured with the media according to the present invention may be prokaryotic cells like bacterial cells or eukaryotic cells like plant or animal cells. The cells can be normal cells, immortalized cells, diseased cells, transformed cells, mutant cells, somatic cells, germ cells, stem cells, precursor cells or embryonic cells, any of which may be established or transformed cell lines or obtained from natural sources.

The size of a particle means the diameter of the particle. If the size of particles is given it means that at least 80 %, preferably at least 90 %, of the particles have the given particle size or are in the given particle size range. The particle diameter is determined by laser light scattering.

An inert atmosphere is generated by filling the respective container or apparatus with an inert gas. Suitable inert gases are noble gases like argon or preferably nitrogen. These inert gases are non-reactive and prevent undesirable chemical reactions from taking place. In the process according to the present invention, generating an inert atmosphere means that the concentration of oxygen is reduced below 10 % (v/v) absolute, e.g. by introducing liquid nitrogen or nitrogen gas.

Different types of mills are known to a person skilled in the art.

A pin mill, also called centrifugal impact mill, pulverizes solids whereby protruding pins on high-speed rotating disks provide the breaking energy. Pin mills are for example sold by Munson Machinery (USA), Premium Pulman (India) or Sturtevant (USA).

A jet mill uses compressed gas to accelerate the particles, causing them to impact against each other in the process chamber. Jet mills are e.g. sold by Sturtevant (USA) or PMT (Austria).

A fitz mill commercialized by Fitzpatrick (USA), uses a rotor with blades for milling.

A process that is run continuously is a process that is not run batchwise. If a milling process is run continuously it means that the media ingredients are permanently and steadily fed into the mill over a certain time.

The cell culture media, especially the full media, according to the present invention typically comprise at least one or more saccharide components, one or more amino acids, one or more vitamins or vitamin precursors, one or more salts, one or more buffer components, one or more co-factors and one or more nucleic acid components.

The media may also comprise sodium pyruvate, insulin, vegetable proteins, fatty acids and/or fatty acid derivatives and/or pluronic acid and/or surface active components like chemically prepared non-ionic surfactants. One example of a suitable non-ionic surfactant are difunctional block copolymer surfactants terminating in primary hydroxyl groups also called poloxamers, e.g. available under the trade name pluronic ^{®} from BASF, Germany.

Saccharide components are all mono- or di-saccharides, like glucose, galactose, ribose or fructose (examples of monosaccharides) or sucrose, lactose or maltose (examples of disaccharides).

Examples of amino acids according to the invention are tyrosine, the proteinogenic amino acids, especially the essential amino acids, leucine, isoleucine, lysine, methionine, phenylalanine, threonine, tryptophan and valine, as well as the non-proteinogenic amino acids, preferably the L-amino acids.

Tyrosine means L- or D- tyrosine, preferably L-tyrosine.

Cysteine means L- or D-cysteine, preferably L-cysteine.

Examples of vitamins are Vitamin A (Retinol, retinal, various retinoids, and four carotenoids), Vitamin B1 (Thiamine), Vitamin B2 (Riboflavin), Vitamin B3 (Niacin, niacinamide), Vitamin B5 (Pantothenic acid), Vitamin B6 (Pyridoxine, pyridoxamine, pyridoxal), Vitamin B7 (Biotin), Vitamin B9 (Folic acid, folinic acid), Vitamin B12 (Cyanocobalamin, hydroxycobalamin, methylcobalamin), Vitamin C (Ascorbic acid), Vitamin D (Ergocalciferol, cholecalciferol), Vitamin E (Tocopherols, tocotrienols) and Vitamin K (phylloquinone, menaquinones). Vitamin precursors are also included.

Examples of salts are components comprising inorganic ions such as bicarbonate, calcium, chloride, magnesium, phosphate, potassium and sodium or trace elements such as Co, Cu, F, Fe, Mn, Mo, Ni, Se, Si, Ni, Bi, V and Zn. Examples are Copper(II) sulphate pentahydrate (CuSO₄5H₂O), Sodium Chloride (NaCl), Calcium chloride (CaCl₂·2H₂O), Potassium chloride (KCI), Iron(II)sulphate, sodium phosphate monobasic anhydrous (NaH₂PO₄), Magnesium sulphate anhydrous (MgSO₄), sodium phosphate dibasic anhydrous (Na₂HPO₄), Magnesium chloride hexahydrate (MgCl₂·6H₂O), zinc sulphate heptahydrate.

Examples of buffers are CO₂/HCO₃ (carbonate), phosphate, HEPES, PIPES, ACES, BES, TES, MOPS and TRIS.

Examples of cofactors are thiamine derivatives, biotin, vitamin C, NAD/NADP, cobalamin, flavin mononucleotide and derivatives, glutathione, nucleotides, phosphates and derivatives.

Nucleic acid components, according to the present invention, are the nucleobases, like cytosine, guanine, adenine, thymine or uracil, the nucleosides like cytidine, uridine, adenosine, guanosine and thymidine, and the nucleotides like adenosine monophosphate or adenosine diphosphate or adenosine triphosphate.

Feed media may have a different composition compared to full media. They typically comprise amino acids, trace elements and vitamins. They might also comprise saccharide components but sometimes for production reasons the saccharide components are added in a separate feed.

A suitable feed medium might for example comprise one or more of the following compounds:

| |
|---|
| L-ASPARAGINE MONOHYDRATE |
| L-ISOLEUCINE |
| L-PHENYLALANINE |
| SODIUM L-GLUTAMATE MONOHYDRATE |
| L-LEUCINE |
| L-THREONINE |
| L-LYSINE MONOHYDROCHLORIDE |
| L-PROLINE |
| L-SERINE |
| L-ARGININE MONOHYDROCHLORIDE |
| L-HISTIDINE MONOHYDROCHLORIDE MONOHYDRATE |
| L-METHIONINE |
| L-VALINE |
| MONO-SODIUM-L-ASPARTATE-MONOHYDRATE |
| L-TRYPTOPHAN |
| CHOLINE CHLORIDE |
| MYO-INOSITOL |
| NICOTINAMIDE |
| CALCIUM-D(+) PANTOTHENATE |
| PYRIDOXINE HYDROCHLORIDE |
| THIAMINE CHLORIDE HYDROCHLORIDE |
| VITAMIN B12 (CYANOCOBALAMINE) MICRONIZED |
| BIOTIN |
| FOLIC ACID |
| RIBOFLAVIN |
| MAGNESIUM SULFATE ANHYDROUS |
| COPPER(II) SULFATE PENTAHYDRATE |
| ZINC SULFATE HEPTAHYDRATE |
| 1,4-DIAMINOBUTANE DIHYDRCHLORIDE |
| AMMONIUM HEPTAMOLYBDATE TETRAHYDRATE |
| CADMIUM SULFATE HYDRATE |
| MANGANESE(II) CHLORIDE TETRAHYDRATE |
| NICKEL(II) CHLORIDE HEXAHYDRATE |
| SODIUM META SILICATE |
| SODIUM METAVANADATE |
| TIN(II) CHLORIDE DIHYDRATE |
| SODIUM SELENITE (ABOUT 45% SE) |
| SODIUM DIHYDROGEN PHOSPHATE MONOHYDRATE |
| AMMONIUM IRON(III) CITRATE (ABOUT 18% FE) |

Freezing according to the present invention means cooling to a temperature below 0°C.

The gist of the present invention is to provide efficient cell culture media, that on the one hand are sufficiently concentrated formulations as required for biomanufacturing processes, e.g. using inline dilution, to reduce the volume of CCM which must be stored in tanks and thus reduce the manufacturing footprint or in general a. to reduce the volume of feed added throughout a fed-batch (FB) process or b. to reduce the CSPR by using concentrated media in continuous processes like perfusion cell culture and thus potentially increase the volumetric titer.

Another key characteristic for amino acid derivatives, in particular when used in media for perfusion application, is that they need to be readily available to cells, to support metabolic requirements. Many amino acid derivatives are not readily bioavailable and require the release of enzymes to cleave them thus releasing the bioavailable canonical amino acid. Consequently, many highly soluble amino acid derivatives may not be suitable for cell culture media.

As shipping and storage of liquid media is more complicated, typically cell culture media are produced as dry powder media which are dissolved in a suitable amount of an aqueous liquid, like water or an aqueous buffer, prior to use.

The simple dissolving of a powdered cell culture medium is often complicated by substances, especially amino acids, which have a poor solubility in aqueous liquids. L-tyrosine for example has a solubility of 0.4 g/L in water at a temperature of 25°C. That means about 0.4 g of L-tyrosine are soluble in 1 liter of water. But the required concentration of tyrosine in cell culture media is often higher.

It has been found that N-norvalyl-L-tyrosine, on the one hand, has a higher solubility in aqueous solutions than tyrosine and on the other hand, can be used as partial or preferably full substitute for tyrosine and is equally suitable as cell culture media component.

The cell culture media according to the present invention can comprise the native tyrosine and salts thereof as well as N-norvalyl-L-tyrosine and salts thereof. In case the media is a feed media, or another media additive, that is added to a basal medium comprising native tyrosine or salts thereof, the said feed media or media additive preferably only comprise N-norvalyl-L-tyrosine or salts thereof but no native tyrosine or salts thereof.

As N-norvalyl-L-tyrosine or salts thereof is equally suitable as tyrosine source as native tyrosine it can be used either as single tyrosine source or as a mixture with other tyrosine derivatives or native tyrosine or salts thereof, whereby there is no need to add native tyrosine or salts thereof to any cell culture medium comprising N-norvalyl-L-tyrosine or salts thereof as N-norvalyl-L-tyrosine or salts thereof can be used as full substitute that is equally bioavailable but has higher solubility.

The overall concentration of N-norvalyl-L-tyrosine in a ready to use liquid basic/perfusion medium and also in a feed medium or medium additive is very flexible. The upper limit is only defined by the solubility of N-norvalyl-L-tyrosine in the respective medium. Consequently, it is typically possible to generate liquid media with a concentration of N-norvalyl-L-tyrosine of up to 100 mmol/L or more, e.g. between 0.1 and 100 mmol/L, preferably between 1 and 60 mmol/L.

To enlarge the solubility of N-norvalyl-L-tyrosine even more, a salt can be formed by reacting N-norvalyl-L-tyrosine with a suitable acid and/or base. The hydrochloride salt is preferred.

The powdered cell culture media of the present invention are preferably produced by mixing all components and milling them. The mixing of the components is known to a person skilled in the art of producing dry powdered cell culture media by milling. Preferably, all components, in dry state, are thoroughly mixed so that all parts of the mixture have nearly the same composition. The higher the uniformity of the composition, the better the quality of the resulting medium with respect to homogenous cell growth. The milling can be performed with any type of mill suitable for producing powdered cell culture media. Typical examples are ball mills, pin mills, fitz mills or jet mills. Preferred is a pin mill, a fitz mill or a jet mill, very preferred is a pin mill.

A person skilled in the art knows how to run such mills.

A large scale equipment mill with a disc diameter of about 40 cm is e.g. typically run at 1-6500 revolutions per minute in case of a pin mill, preferred are 1-3000 revolutions per minute.

The milling can be done under standard milling conditions resulting in powders with particle sizes between 10 and 300 µm, most preferably between 25 and 120 µm.

Preferably, all components of the mixture which is subjected to milling are dry. This means, if they comprise water, they do only comprise water of crystallization but not more than 10 %, preferably not more than 5 % most preferred not more than 2 % by weight of unbound or uncoordinated water molecules.

In a preferred embodiment, the milling is performed in an inert atmosphere. Preferred inert protective gas is nitrogen.

In another preferred embodiment, all components of the mixture are freezed prior to milling. The freezing of the ingredients prior to the milling can be done by any means that ensures a cooling of the ingredients to a temperature below 0°C and most preferably below - 20°C. In a preferred embodiment the freezing is done with liquid nitrogen. This means the ingredients are treated with liquid nitrogen, for example by pouring liquid nitrogen into the container in which the ingredients are stored prior to introduction into the mill. In a preferred embodiment, the container is a feeder. If the container is a feeder the liquid nitrogen is preferably introduced at the side or close to the side of the feeder at which the ingredients are introduced.

Typically the ingredients are treated with the liquid nitrogen over 2 to 20 seconds.

Preferably the cooling of the ingredients is done in a way that all ingredients that enter into the mill are at a temperature below 0°C, most preferred below - 20°C.

In a preferred embodiment, all ingredients are put in a container from which the mixture is transferred in a feeder, most preferred in a metering screw feeder. In the feeder the ingredients are sometimes further mixed - depending on the type of feeder - and additionally cooled. The cooled mixture is then transferred from the feeder to the mill so that the mixture which is milled in the mill preferably still has a temperature below 0°C, more preferred below - 20 °C.

Typically the blending time, that means the residence time of the mixture of ingredients in the feeder is more than one minute, preferably between 15 and 60 minutes.

A metering screw feeder, also called dosage snail, is typically run at a speed of 10 to 200 revolutions per minute, preferably it is run at 40 to 60 revolutions per minute.

Typically, the temperature of the mill is kept between -50 and +30°C. In a preferred embodiment, the temperature is kept around 10°C.

The oxygen level during milling preferably is below 10 % (v/v).

The process can be run e.g. batch-wise or continuously. In a preferred embodiment the process according to the present invention is done continuously by, over a certain time, permanently filling the mixture of ingredients into a feeder for cooling and permanently filling cooled mixture from the feeder into the mill.

After milling, the resulting dry powder medium might be further compacted to enlarge the size of the particles, e.g. by dry compaction in a roll press.

For use of the powdered media aqueous liquid, preferably water (most particularly distilled and/or deionized water or purified water or water for injection) or an aqueous buffer is added to the media and the components are mixed until the medium is totally dissolved in the solvent and the ready to use liquid medium is generated.

The aqueous liquid may also comprise saline, soluble acid or base ions providing a suitable pH range (typically in the range between pH 1.0 and pH 10.0), stabilizers, surfactants, preservatives, and alcohols or other polar organic solvents.

It is also possible to add further substances like buffer substances for adjustment of the pH, fetal calf serum, sugars etc. to the mixture of the cell culture medium and the solvent. The resulting liquid cell culture medium is then contacted with the cells to be grown or maintained.

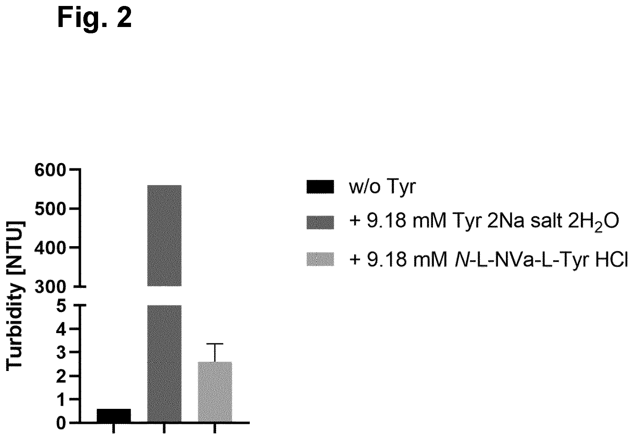

While media compositions comprising a higher concentration tyrosine would show turbidity when mixed with the aqueous liquid due to non-dissolved tyrosine, the cell culture media according to the present invention in which tyrosine is partly or preferably fully substituted by N-norvalyl-L-tyrosine give clear solutions, as shown by the turbidity measurement in Example 2 and Figure 2.

In addition, it has been found that liquid media comprising N-norvalyl-L-tyrosine instead of tyrosine are more stable when stored over a longer period of time. While liquid media comprising tyrosine show a decrease in tyrosine concentration already after about 20 days, media comprising N-norvalyl-L-tyrosine are stable for more than 50, typically more than 70 days. Preferably the media are stored protected from light at room temperature or below, e.g. between 4°C and RT. The present invention is thus also directed to liquid cell culture media which comprises N-norvalyl-L-tyrosine but no tyrosine and which are stable and do not show a decomposition of N-norvalyl-L-tyrosine over 50 days, preferably over 70 days.

The present invention is further directed to a process for culturing cells by
a) providing a bioreactor
b) mixing the cells to be cultured with a liquid cell culture medium according to the present invention
c) incubating the mixture of step b)

Optionally the liquid cell culture medium is produced by dissolving a dry powder medium comprising N-norvalyl-L-tyrosine in an aqueous liquid.

In one embodiment the bioreactor is a perfusion bioreactor.

A bioreactor is any vessel or tank in which cells can be cultured. Incubation is typically done under suitable conditions like suitable temperature etc. A person skilled in the art is aware of suitable incubation conditions for supporting or maintaining the growth/culturing of cells.

A perfusion bioreactor is a bioreactor in which perfusion cell culture can be performed. It comprises the bioreactor vessel which is typically closed during cell culture, a stirrer in the vessel, a line for introducing fresh medium, a harvest line for removing the harvest stream comprising cells, liquid medium and target product from the bioreactor and a cell retention device in the harvest line that retains the cells while the liquid part of the harvest can be collected. A review about perfusion cell culture providing details about favorable set ups can be found in "Perfusion mammalian cell culture for recombinant protein manufacturing - A critical review" Jean-Marc Bielser et al., Biotechnology Advances 36 (2018) 1328-1340.

The present invention is thus also directed to a process for perfusion cell culture, comprising culturing cells in a perfusion bioreactor with a media inlet and a harvest outlet whereby
i. continuously or one or several times during the cell culture process new cell culture medium comprising N-norvalyl-L-tyrosine is inserted into the bioreactor via the media inlet
ii. continuously or one or several times during the cell culture process harvest is removed from the bioreactor via the harvest outlet.

The cell culture medium added in step i. can have the basic concentration equivalent to the cell culture medium in the bioreactor or it can be concentrated.

In addition, in one embodiment, a second cell culture medium, preferably a concentrated medium which comprises only fewer components compared to the medium in the bioreactor and the medium inserted in step i. is added one or several times during the cell culture process to the bioreactor via the media inlet or an additional inlet, whereby the second cell culture medium comprises N-norvalyl-L-tyrosine. Preferably the addition of the second cell culture medium is done at least 50% of the time, preferably at least 75% of the time during perfusion phase without increasing the overall VVD. This means if the second cell culture medium is inserted into the bioreactor with a certain VVD, the VVD of the basal medium is preferably at least reduced by the VVD of the second medium.

The performance of a cell culture perfusion process is known to the skilled person. Typically this is done by inoculating the bioreactor with a basal medium and cells. Inoculation cell density is typically between 0.5 and 10 mio cells/mL, preferably between 0.5 and 1.0 mio cells/mL.

It is possible to run the process in perfusion mode directly from the beginning, i.e. directly after inoculation with the cells. But the process is preferably first run in batch mode for some time while the number of cells, the VCD, increases. Typically, the process is run in batch mode for 2 to 8 days, preferably 3 to 5 days and then perfusion is started. In any case, perfusion is preferably turned on before growth stops being exponential. This can for example be evaluated in prior cell line characterization experiments.

Perfusion is then turned on by typically setting a constant perfusion rate and a constant bleed rate to reach a constant VCD. But it is also possible to run the perfusion cell culture as dynamic perfusion whereby the VCD is not constant. The skilled person is aware of various way to run a perfusion cell culture.

It has been found that the present invention is also very suitable for the preparation of feed media. Due to the limitations in the availability of certain amino acids especially in the concentrations necessary for feed media the feed media cannot be prepared in the desired high concentrations or they need to be prepared under drastic pH condition like very basic pH. This might negatively affect the nutrition supply to cells and to some extent accelerate cell death by exposure to extreme basic pH values.

Consequently here is a need for feed media that comprise all needed components in one feed and at high concentrations. In addition the pH of the feed should not negatively influence the cell culture.

It has been found that N-norvalyl-L-tyrosine has an improved solubility and can be used in highly concentrated feed without any negative effect and sometimes even positive effect on the cell growth and/or productivity at a pH below 8.5.

The present invention is thus also directed to a feed medium either in form of a powdered medium or after dissolution in form of a liquid medium.

The resulting liquid medium comprises N-norvalyl-L-tyrosine in concentrations typically above 10 mmol/L or even above 60 mmol/L, preferably between 10 and 60 mmol/L, and preferably has a pH of 8.5 or less. In a preferred embodiment, the pH is between 6.5 and 7.8. Preferably the feed medium does not comprise tyrosine.

The present invention is also directed to a process for culturing cells in a bioreactor by
- Filling into a bioreactor cells and an aqueous cell culture medium
- Incubating the cells in the bioreactor
- Adding a cell culture medium to the bioreactor, continuously over the whole time or once or several times within the cells incubation time
whereby the cell culture medium that is added preferably has a pH of less than pH 8.5 and comprises N-norvalyl-L-tyrosine. Typically the medium comprises between 15 and 150 g/L , preferably 25 to 150 g/L of solid ingredients that are dissolved in the aqueous liquid.

In one embodiment the medium that is added is a feed medium and the process is a fed batch process. In another medium the medium that is added is a perfusion medium and the process is a perfusion process.

It has been found that by N-norvalyl-L-tyrosine a feed medium can be obtained that comprises all necessary feed components at high concentrations (overall concentration between 100 and 250 g/L). In contrast to known processes where two or more different feed media need to be fed to the bioreactor, the present invention provides a medium and a method which enables the use of one feed medium that comprises all components in high concentrations. In addition the pH of the feed medium according to the present invention typically is below 8.5.

In a preferred embodiment, in the process of the present invention the feed medium that is added during the incubation either continuously or once or several times within said time to the bioreactor always has the same composition.

It has been found that the cell culture processes using N-norvalyl-L-tyrosine have a performance equal to the processes with tyrosine. The quality of the proteins generated with cell culture processes using N-norvalyl-L-tyrosine is equivalent to the quality of processes with tyrosine. Due to the higher solubility of N-norvalyl-L-tyrosine, its excellent bioavailability and the improved stability of N-norvalyl-L-tyrosine in liquid media compared to tyrosine, N-norvalyl-L-tyrosine is not only a full substitute of tyrosine in cell culture media but provides more flexibility and advantages compared to tyrosine or salts thereof.

The present invention is further illustrated by the following figures and examples, however, without being restricted thereto.

### Examples

The following examples represent practical applications of the invention.

### Example 1: N-L-norvalyl-L-tyrosine HCl shows an increased solubility compared to the canonical amino acid L-Tyrosine and salts thereof in water

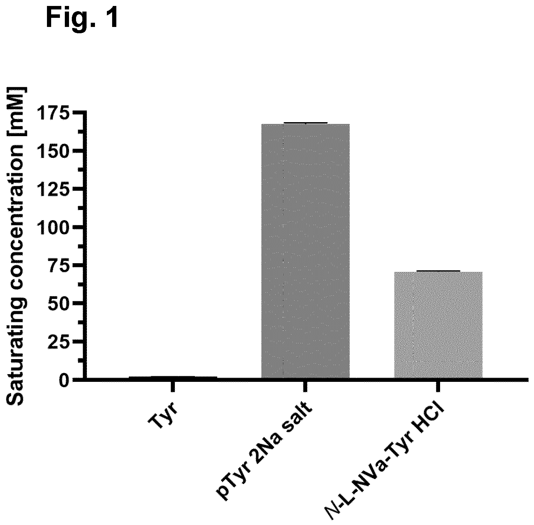

The maximum solubility of N-L-norvalyl-L-tyrosine HCl (*N*-L-NVa-Tyr HCl) was compared with the solubility of canonical amino acid L-Tyrosine (Tyr) and salts thereof in water at 25°C and pH 7.3±0.1 through the preparation of an oversaturated solution. Measurement was performed after a filtration step (0.22µm) to remove undissolved compound. The solution was measured by amino acid analysis using ultra performance liquid chromatography (UPLC). As shown in Figure 1, Tyr showed a maximal solubility of 2.20±0.02 mM. PTyr (Ophospho-tyrosine) 2Na salt exhibited a max solubility of 167.5±0.8 mM at pH 7.3 whereas *N*-L-NVa-Tyr HCl showed a max solubility of 70.7±0.6 mM

### Example 2: N-L-norvalyl-L-tyrosine HCl shows an increased solubility compared to the canonical amino acid L-Tyrosine and salts thereof in 6-fold concentrated CCM

The solubility of Tyr 2Na salt 2H₂O was compared with the solubility of its respective derivatives or salts thereof in 6-fold concentrated CCM (modified EX-CELL^{®} Advanced HD Perfusion Medium) at pH 7.3±0.1 and 25°C by turbidity measurement.

As shown in Figure 2, the turbidity of *N*-L-NVa-Tyr HCl was much lower when compared to the turbidity of Tyr 2Na salt 2H₂O in 6-fold concentrated CCM at neutral pH. For *N*-L-NVa-Tyr HCl, the decrease in turbidity was more than 95 %. Altogether, these results indicate that N-L-norvalyl-L-tyrosine and its salts are suitable alternatives to increase solubility of cell culture media and feed formulations by replacement of L-Tyrosine disodium salt dihydrate.

### Example 3: N-L-norvalyl-L-tyrosine HCl has an increased solubility compared to its respective amino acid L-Tyrosine and salts thereof in CCM

The maximum solubility of Tyr 2Na salt 2H₂O was compared with the solubility of its respective derivatives or salts thereof in 6-fold concentrated CCM (modified EX-CELL^{®} Advanced HD Perfusion Medium) at pH 7.3±0.1 and 25°C through the preparation of an oversaturated derivative solution. Samples were taken after 30 minutes of stirring. Measurement was performed after a filtration step (0.22µm) to remove undissolved compound. The concentration of Tyr 2Na salt 2H₂O and *N*-L-NVa-Tyr HCl was determined by amino acid analysis using UPLC.

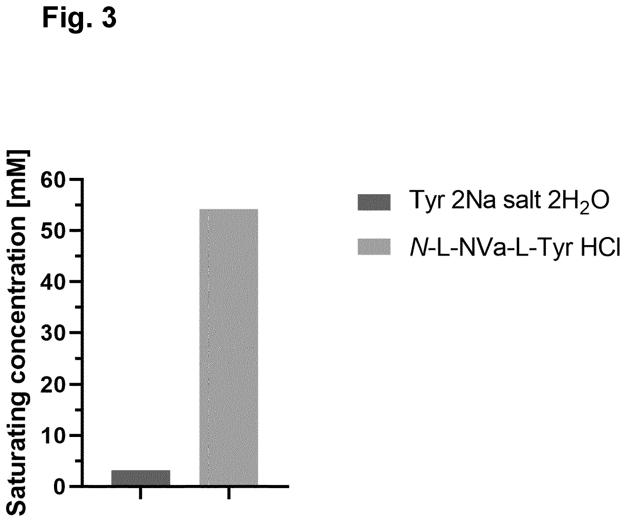

The maximum solubility of Tyr 2Na salt 2H₂O in modified EX-CELL^{®} Advanced HD Perfusion Medium depleted in Tyr was found to be approximately 3 mM, whereas for *N*-L-NVa- Tyr HCl, a maximum solubility of approximately 54 mM was detected (Figure 3). This indicates that N-L-Norvalyl-Tyr HCl is at least 18 times more soluble than Tyr 2Na salt 2H₂O in modified EX-CELL^{®} Advanced HD Perfusion Medium depleted in Tyr.

### Example 4: 6-fold concentrated CCM containing N-L-norvalyl-L-tyrosine HCl has an increased stability compared to CCM containing L-Tyrosine and salts thereof. It is stable at 4°C, and also moderately stable at RT, light protected for a period of three months.

Stability studies were performed to monitor the stability of medium containing Tyr 2Na salt 2H₂O and its respective derivative *N*-L-NVa-Tyr or salts thereof at 4°C and room temperature. Therefore, a 6-fold concentrated CCM (modified EX-CELL^{®} Advanced HD Perfusion Medium depleted in Tyr) at pH 7.3±0.1 was prepared with 6-fold concentration of Tyr 2Na salt 2H₂O and N-L-NVa-Tyr HCl and stored light protected for three months. The concentration of Tyr, *N*-L-NVa-Tyr HCl and NVa was determined by amino acid analysis using UPLC. To remove undissolved compound, a filtration step (0.22 µm pore size) was performed prior to amino acid analysis.

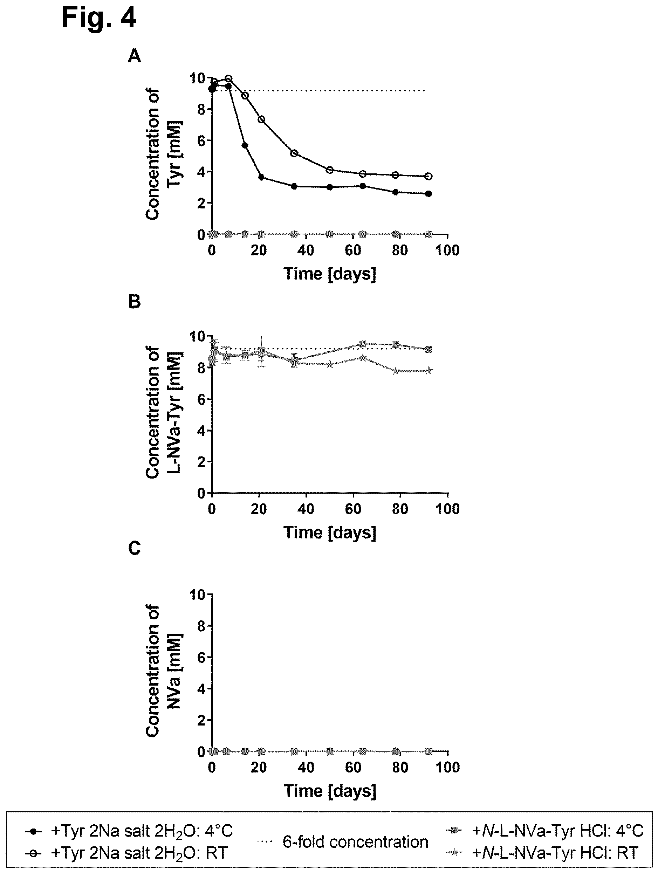

Whereas modified EX-CELL^{®} Advanced HD Perfusion Medium depleted in Tyr supplemented with Tyr 2Na salt 2H₂O was found to be unstable at 4°C and RT, the concentration of *N*-L-NVa-Tyr HCl was constant for 92 days at 4°C. No free Tyr nor NVa was detected. At RT, CCM containing *N*-L-NVa-Tyr HCl was found to be stable for 78 days as shown in Figure 4. Afterwards, the concentration of *N*-L-NVa-Tyr HCl decreased slightly by 10 % over the three months of storage at room temperature. However, neither free Tyr nor NVa was detected.

This indicates that 6-fold concentrated CCM containing *N*-L-NVa-Tyr HCl is stable if stored for three months at 4°C and RT protected from light. 6-fold concentrated CCM containing Tyr 2Na salt 2H₂O was stable for six days at 4°C and 14 days at RT.

### Example 5: N-L-norvalyl-L-tyrosine HCl can replace its respective amino acid Tyr 2Na salt 2H₂O. Cell culture results with a CHOK1 GS clone A producing an IgG1.

For cell culture experiments, a CHOK1 GS suspension cell line expressing a human IgG1, was used. Cells were cultivated in triplicates or quadruplicates in modified EX-CELL^{®} Advanced HD Perfusion Medium (Merck Darmstadt, Germany) using 50 mL spin tubes with a starting culture volume of 30 mL and a seeding density of 0.3x10⁶ viable cells/mL. Incubation was carried out at 37°C, 5% CO₂, 80 % humidity and an agitation of 320 rpm. The Tyr derivatives were added in the CCM (modified EX-CELL^{®} Advanced HD Perfusion Medium depleted in Tyr) instead of the amino acid Tyr. The pH of the CCM was neutral (pH 7.3±0.1). The positive control contained Tyr 2Na salt 2H₂O whereas the negative control contained pTyr 2Na salt, a tyrosine derivative that is not readily bioavailable. Besides, Pro-Tyr, Gly-Tyr and Tyr-His were also tested in comparison to *N*-L-NVa-Tyr HCl. Experiments for Gly-Tyr and *N*-L-NVa-Tyr HCl were repeated at least 3 times, as well as for positive and negative control.

Viable cell density (VCD = viable cells/mL) and viability were evaluated with a Vi-CELL XR (Beckman Coulter, Fullerton, CA). Metabolite concentrations were monitored using a Cedex Bio HT (Roche Diagnostics, Mannheim, Germany) based on spectrophotometric and turbidometric methods. Quantification of amino acids was carried out via UPLC after derivatization with the AccQ•TagUltra^{®} reagent kit. Derivatization, chromatography and data analysis were carried out following the supplier recommendations (Waters, Milford, MA).

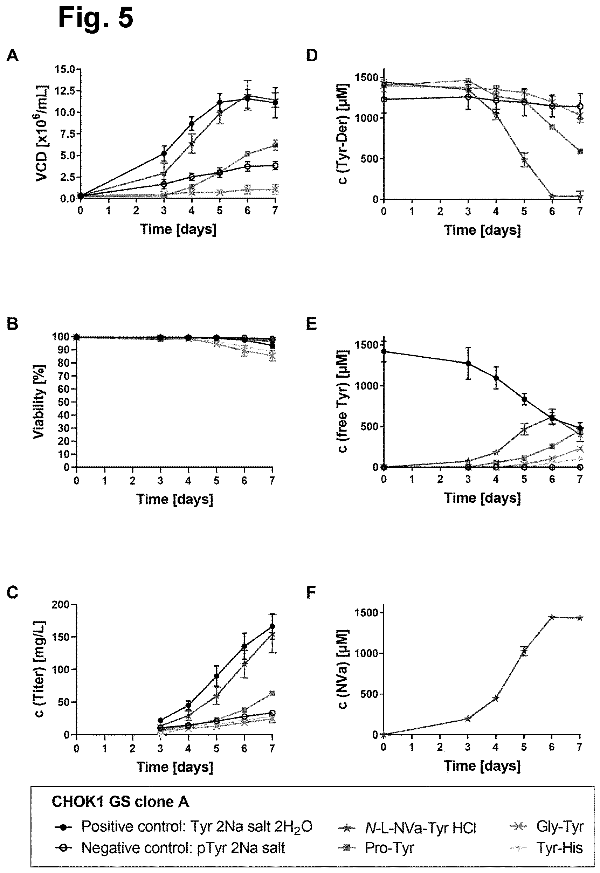

Figure 5 displays the cell performance of CHOK1 GS clone A during a seven days batch process. VCD and IgG concentration were comparable to the positive control Tyr 2Na salt 2H₂O when adding *N*-L-NVa-Tyr HCl to modified EX-CELL^{®} Advanced HD Perfusion Medium depleted in Tyr (Figure 5). Both conditions reached a maximum VCD of 12±1 x10⁶ viable cells/mL after six days in culture (Figure 5A) and resulted in a titer production of approximately 160 mg/L after seven days. Also, viability was constantly above 90 % for N-L-NVa-Tyr HCl (Figure 5B)Fehler! **Es wurde kein Textmarkenname vergeben..** A decrease of VCD by45 % and titer by over 60 % was observed for Pro-Tyr compared to the positive control Tyr 2Na salt 2H₂O (Figure 5C). The negative control pTyr 2Na salt reached a maximum VCD of 3.8±0.5 x10⁶ and maximum IgG concentration of 34±3 mg/L after seven days in culture. Overall, Gly-Tyr and Tyr-His showed a lower cell performance compared to the negative control pTyr 2Na salt.

The concentration of free Tyr and TyrDer was determined in spent media (Figure 5D+E). Since the formation of free Tyr from N-L-N L-NVa-Tyr HCl a-Tyr HCl is also releasing Norvaline (NVa), this amino acid was monitored in the supernatant over the duration of the batch process as well (Figure 5F). In the condition in which Tyr 2Na salt 2H₂O was replaced by *N*-L-NVa-Tyr HCl, the concentration of *N*-L-NVa-Tyr HCl decreased from day three to six by over 97 % (Figure 5D). The concentration of free Tyr (Figure 5E), and NVa (Figure 5F) increased whereas the concentration of *N*-L-NVa-Tyr HCl decreased accordingly.

### Batch experiments with different CHO cell lines

The applicability of *N*-L-NVa-yr HCl for different bioprocesses was demonstrated by performing batch experiments with different CHO cell lines (1x CHOK1 non-GS, 5x CHOZN GS) producing either an IgG1 or a fusion protein.

Therefore, cells were cultivated in triplicates or quadruplicates in modified EX-CELL^{®} Advanced HD Perfusion Medium (Merck Darmstadt, Germany) using 50 mL spin tubes with a starting culture volume of 30 mL and a seeding density of 0.3x10⁶ viable cells/mL. Incubation was carried out at 37°C, 5% CO₂, 80 % humidity and an agitation of 320 rpm. The Tyr derivatives were added in the CCM (modified EX-CELL^{®} Advanced HD Perfusion Medium depleted in Tyr) instead of the canonical amino acid Tyr. The pH of the CCM was neutral (pH 7.3±0.1). The positive control contained Tyr 2Na salt 2H₂O whereas the negative control contained pTyr 2Na salt. No negative control was used for CHOK1 non-GS clone.

Viable cell density (VCD = viable cells/mL) and viability were evaluated with a Vi-CELL XR (Beckman Coulter, Fullerton, CA). Metabolite concentrations were monitored using a Cedex Bio HT (Roche Diagnostics, Mannheim, Germany) based on spectrophotometric and turbidimetric methods. Quantification of amino acids was carried out via UPLC after derivatization with the AccQ•TagUltra^{®} reagent kit. Derivatization, chromatography and data analysis were carried out following the supplier recommendations (Waters, Milford, MA).

### Example 6: Confirmation of N-L-Norvalyl-Tyr HCl performance with a CHOK1 non-GS producing an IgG1.

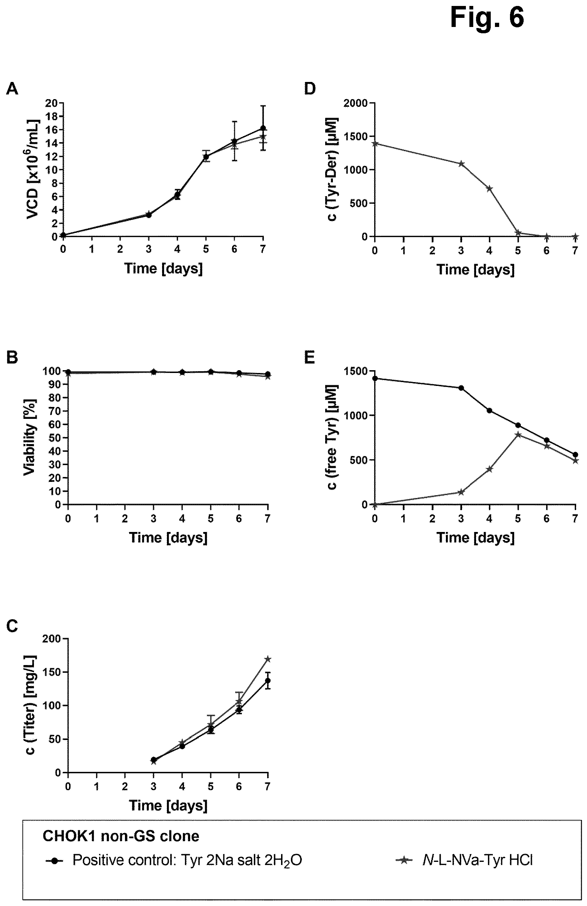

Figure 6 displays the cell performance of a CHOK1 non-GS clone, producing an IgG1 in a batch experiment. Overall, results indicate a similar cell performance for *N*-L-NVa-Tyr HCl compared to the positive control Tyr 2Na salt 2H₂O (Figure 6 A+B). Both conditions reached a maximum VCD in the range of 15-16 x10⁶ viable cells/mL after seven days in culture and viability was constantly above 95 %. *N*-L-NVa-Tyr HCl resulted in a slightly increased titer production by 23 % (Figure 6C). The concentration of *N*-L-NVa-Tyr HCl of 1513 µM was fully depleted after six days in culture (Figure 6D), whereas the concentration of Tyr increased to a maximum of 783.5 µM on day five and afterwards decreased similar to the positive control (Figure 6E).

### Example 7: Confirmation of N-L-Norvalyl-Tyr HCl performance with a CHOZN GS clone A producing a fusion protein.

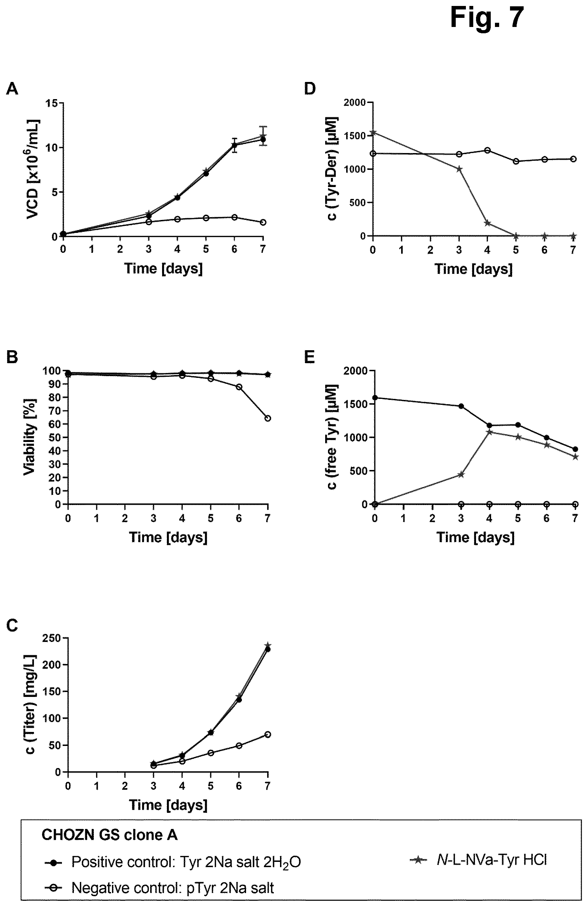

Results of a CHOZN GS clone A, producing a fusion protein indicate a similar cell performance for *N*-L-NVa-Tyr HCl compared to the positive control Tyr 2Na salt 2H₂O (Figure 7 A-C). Both conditions reached a maximum VCD of approximately 11.0 x10⁶ viable cells/mL and a titer production in the range of 230 mg/L on day seven. The viability was constantly above 97 %. *N*-L-NVa-Tyr HCl was fully depleted after five days in culture (Figure 7D), while the concentration of free Tyr increased from 0 µM on day zero to a maximum of 1080 µM on day four and afterwards decreased similarly to the positive control (Figure 7E). In contrast, the negative control pTyr 2Na salt maintained its initial concentration and no free Tyr was detected.

### Example 8: Confirmation of N-L-Norvalyl-Tyr HCl performance with a CHOZN GS clone B producing a fusion protein.

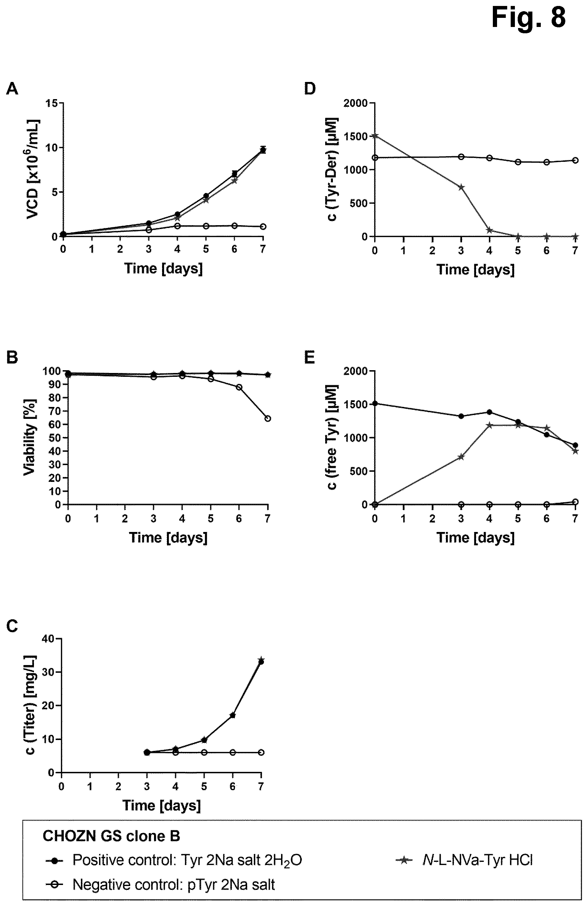

Like CHOZN GS clone A, CHOZN GS clone B, also producing a fusion protein shows a similar cell performance for *N*-L-NVa-Tyr HCL as for the positive control (Figure 8A-C). The maximum VCD was for both conditions in the range of 9-10 x10⁶ viable cells/mL and titer production at 33 mg/L on day seven of culture. Viability was for both conditions constantly above 97 % (Figure 8B).

*N*-L-NVa-Tyr HCl was decreased by over 90 % after four days in culture (Figure 8D), while the concentration of free Tyr increased from 0 µM on day zero to a maximum of 1187 µM on day four and afterwards decreased similarly to the positive control (Figure 8E). In contrast, the negative control pTyr 2Na salt maintained its initial concentration and no or less free Tyr was detected.

### Example 9: Confirmation of N-L-Norvalyl-Tyr HCl performance with a CHOZN GS clone C producing an IgG1.

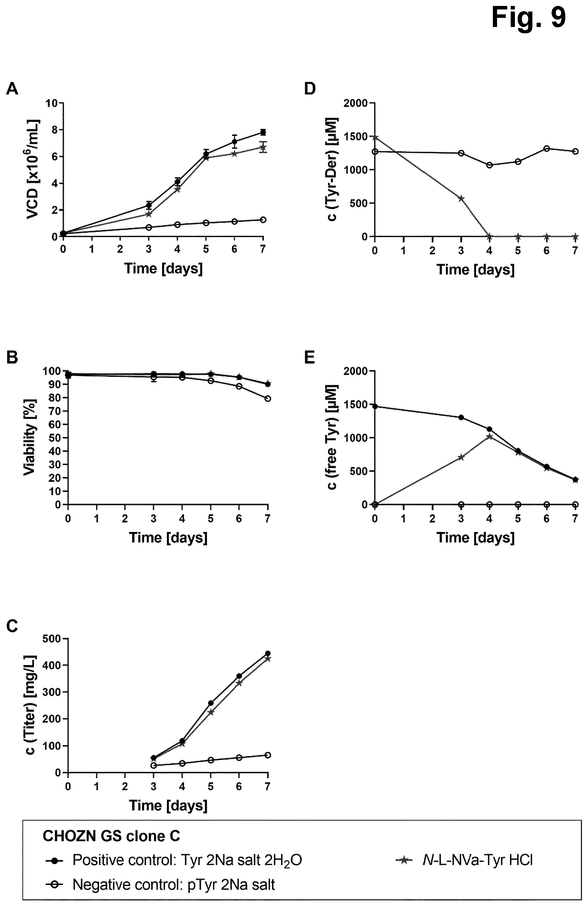

Figure 9 displays the cell performance of a CHOZN GS clone C, producing an IgG1 in a batch experiment. Overall results indicate a similar cell performance for *N*-L-NVa-Tyr HCl compared to the positive control Tyr 2Na salt 2H₂O (Figure 9A-C). Both conditions reached a maximum VCD in the range of 6.5-8 x10⁶ viable cells/mL and a titer production in the range of 430 mg/L after seven days in culture. Viability was constantly above 95 % until day six. The concentration of *N*-L-NVa-Tyr HCl of 1513 µM was fully depleted after four days in culture (Figure 9D), whereas the concentration of Tyr increased to a maximum of 1017 µM on day four and afterwards decreased similarly to the positive control Figure 9E).

### Example 10: Confirmation of N-L-Norvalyl-Tyr HCl performance with a CHOZN GS clone D producing an IgG1.

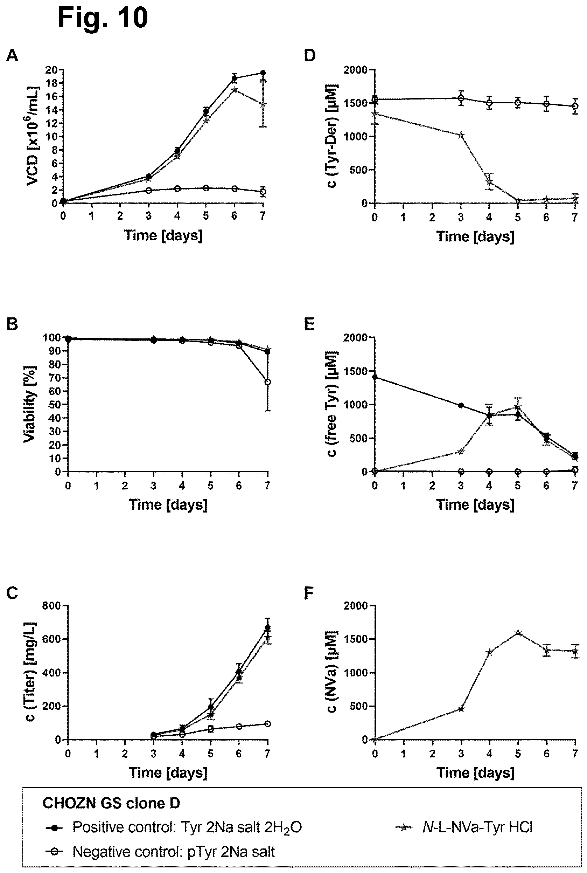

Results of a CHOZN GS clone D, producing an IgG1 indicate a similar cell performance for *N*-L-NVa-Tyr HCl compared to the positive control Tyr 2Na salt 2H₂O (Figure 10A-C). The positive control reached a maximum VCD of 20±1 x10⁶ viable cells/mL and a titer of 668±55 mg/L after seven days in culture. *N*-L-NVa-Tyr HCl resulted in a maximum VCD of 16.9±0.5 x10⁶ on day six and a titer of 609±39 mg/L after seven days.

The viability was constantly above 95 % until day six. *N*-L-NVa-Tyr HCl was decreased by over 97 % after five days in culture (Figure 10D), while the concentration of free Tyr increased from 0 µM on day zero to a maximum of 968±133 µM on day five and afterwards decreased similarly to the positive control (Figure 10E). The concentration of NVa increased to a maximum of 1590 µM on day five, and was afterwards maintained in this range (Figure 10F). In contrast, the negative control pTyr 2Na salt maintained its initial concentration and no free Tyr was detected.

### Example 11: Confirmation of N-L-Norvalyl-Tyr HCl performance with a CHOZN GS clone E producing an IgG1.

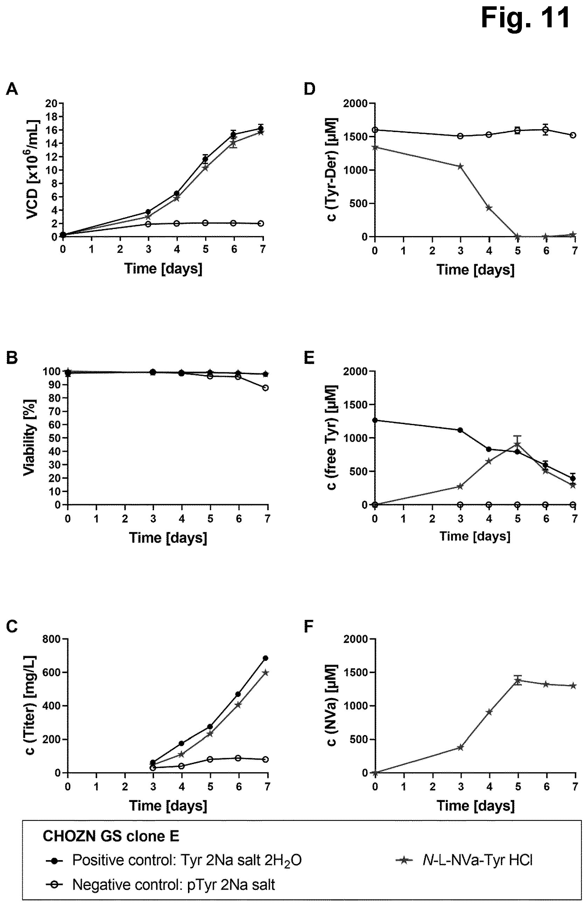

Figure 11 displays the cell performance of a CHOZN GS clone E, producing an IgG1 in a batch experiment. Overall results indicate a similar cell performance for *N*-L-NVa-Tyr HCl compared to the positive control Tyr 2Na salt 2H₂O (Figure 11A-C).

Both conditions reached a maximum VCD in the range of 16 x10⁶ viable cells/mL on day seven. The positive control resulted in a titer of 685±5 mg/L and for *N*-L-NVa-Tyr HCl a titer of 597±8 mg/L was reached after seven days in culture. Viability was constantly above 98 %.

The concentration of *N*-L-NVa-Tyr HCl of 1513 µM was fully depleted after five days in culture (Figure 11D), whereas the concentration of free Tyr increased to a maximum of 911±120 µM on day five and afterwards decreased similarly to the positive control (Figure 11E). The NVa increase was inversely proportional to the decrease in *N*-L-NVa-Tyr HCl up to the NVa concentration of 1300 µM, which was roughly maintained from day five on (Figure 11F). In contrast, the negative control maintained a VCD at approximately 2.0 x10⁶ viable cells/mL, reached a maximum titer below 100 mg/L, its initial concentration was maintained and no free Tyr was detected.

### Example 12: Quality of antibodies

The quality of the antibody produced in the control batch process using CHOK1 GS clone A and CHOZN GS clone D (CCM containing Tyr 2Na salt 2H₂O) was compared to the quality of the antibody produced with CCM depleted in Tyr and supplemented with *N*-L-NVa-Tyr HCl.

The antibody was purified from the cell culture supernatant using protein A PhyTips^{®} (PhyNexus Inc, San Jose, CA). 15 µg purified protein was subjected to released N-glycan analysis. Samples were prepared according to the instructions of the GlycoWorks^{™} RapiFluor-MS^{™} N-Glycan Kit. After sample preparation, glycans were stored in the autosampler at 4°C and separated on an ACQUITY UPLC with Glycan BEH Amide column (2,1 x 150 mm, Waters Art-Nr. 186004742) heated to 45°C. Injection volume was 18µL. Separation was performed according to below gradient of Eluent A (50mM ammonium formate pH 4.4) and Eluent B (100% ACN):

**Table 1: Method of glycan separation**

| **Time [min]** | **Eluent A [%]** | **Eluent B [%]** | **Flow rate [mL/min]** |
|---|---|---|---|
| **0** | 20 | 80 | 0.5 |
| **3** | 20 | 80 | 0.5 |
| **6** | 27 | 73 | 0.5 |
| **58** | 43 | 57 | 0.5 |
| **59.5** | 100 | 0 | 0.2 |
| **62.5** | 100 | 0 | 0.2 |
| **66.1** | 20 | 80 | 0.2 |
| **70.6** | 20 | 80 | 0.5 |
| **78** | 20 | 80 | 0.5 |

Glycan species were quantified using fluorescence detection at 425nm after excitation at 265nm. Glycans were identified by mass using Synapt G2 HDMS.

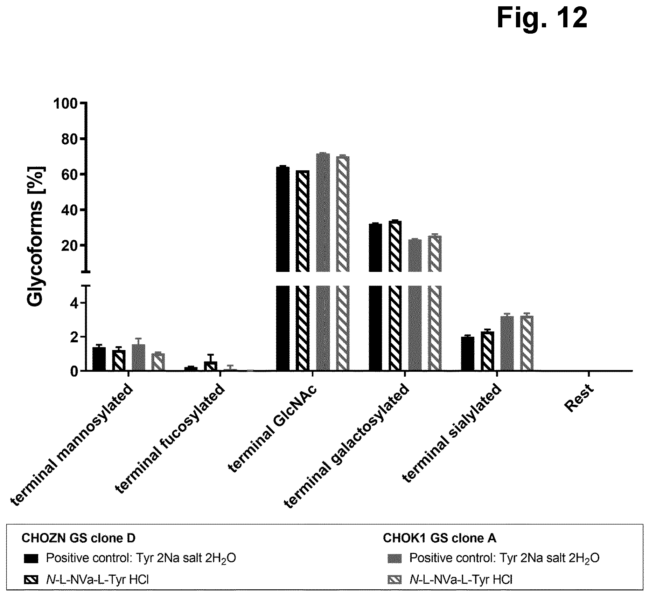

Results obtained for glycosylation (Figure 12) indicate no difference between the control condition Tyr 2Na salt 2H₂O and the condition where Tyr was exchanged by *N*-L-NVa-Tyr HCl. Hence, the amino acid exchange has no impact on the glycosylation pattern of IgG produced in this study.

Antibody aggregation was measured using size exclusion chromatography on an Water Acquity UPLC system using a TSKgel SuperSW3000 column (Tosoh Bioscience). The mobile phase was 0.05 M Sodium phosphate, 0.4 M Sodium perchlorate, pH 6.3 and the flow rate was 0.35 mL/min. The sample concentration was adjusted to 1.0 mg/mL after the IgG purification, using the storage buffer and the detection was performed using the absorbance at 214 nm.

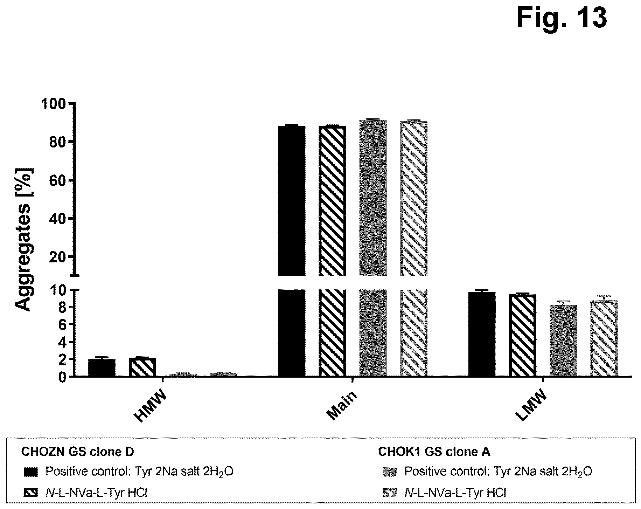

Results obtained for aggregation (Figure 13) indicate no difference between the control condition Tyr 2Na salt 2H₂O and the condition where Tyr was exchanged by *N*-L-NVa-Tyr HCl. Hence, the amino acid exchange has no impact on the aggregation of IgG produced in this study.

Antibody fragmentation was measured on a Capillary Electrophoresis CESI 8000 (Beckman Coulter/Sciex) using CE-SDS according to the manufacturer's instructions. 100 µg purified antibody sample was used in a total volume of 45 µL storage buffer. The samples were not alkylated. Prior to measurement, the samples were mixed with an internal standard (10 kDa) and SDS sample buffer, followed by an incubation step at either 70°C, 500 rpm for 5 min for the IgG produced by CHOK1 GS clone A or at 60°C, 500 rpm for 10 min for the IgG produced by CHOZN GS clone D.

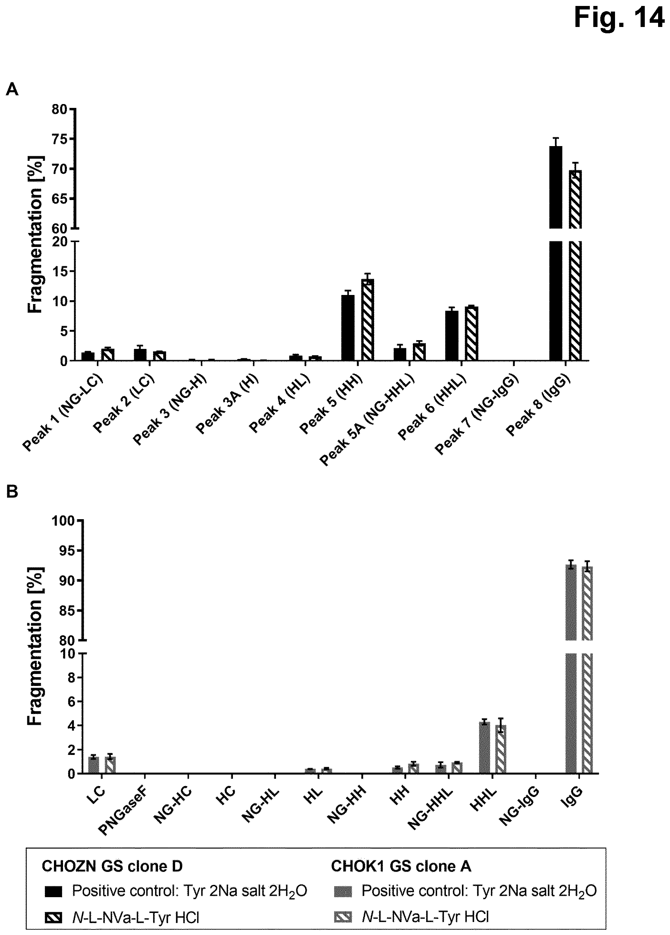

Results obtained for fragmentation (Figure 14) indicate no difference between the control condition Tyr 2Na salt 2H₂O and the condition where Tyr was exchanged by *N*-L-NVa-Tyr HCl. Hence, the amino acid exchange has no impact on the fragmentation of IgG produced in this study.

Apostol et al. reported that Norvaline can be incorporated instead of leucine in the α and β subunits of recombinant human hemoglobin expressed in *Escherichia coli* (Apostol, I., Levine, J., Lippincott, J., Leach, J., Hess, E., Glascock, C. B., Weickert, M. J., & Blackmore, R. (1997). Incorporation of norvaline at leucine positions in recombinant human hemoglobin expressed in Escherichia coli. Journal of Biological Chemistry, 272(46), 28980-28988. https://doi.org/10.1074/jbc.272.46.28980). Norvaline exhibit comparable electric charges as Leucine, but is smaller and less hydrophobic. Putative incorporation of NVa instead of Leu can be studied via intact mass analysis and peptide mapping.

### Middle-up analysis of recombinant mAb after reduction

Molecular masses of mAb light and heavy chains obtained after reduction were measured using an UHPLC (Vanquish Horizon UHPLC, Thermo Fisher Scientific) coupled with an ESI-Q-ToF mass spectrometer (Impact II, Bruker Daltonics). Briefly, 500 ng of samples were loaded on an Reversed-Phase column (1000Å, 5µm, 2.1x50mm, Agilent) thermostated at 80°C with a flow rate of 0.6 mL/min and eluted with the gradient presented in Table 2. LC-MS gradient used for intact mass analysis. MS analysis was performed using the Impact II mass spectrometer equipped with an ESI source (Bruker Daltonics). MS acquisition was performed in positive mode with end plate offset and capillary voltages set at 500 and 4500 V, respectively. Nebulizer and dry gas were set at 3.0 bar and 12.0 L/min, respectively. MS spectrum was acquired over the m/z range 300-3000 with a scan rate of 1 Hz. Calibration was carried out using the internal lockmass at 1221.9906. Charge state deconvolution was performed using the maximum entropy algorithm.

**Table 2. LC-MS gradient used for intact mass analysis.**

| Buffer A: 0.1% FA; Buffer B: 0.1% FA in acetonitrile | | | | | | |
|---|---|---|---|---|---|---|
| **Time (min)** | **0** | **10.0** | **10.1** | **11.0** | **11.1** | **16** |
| **Flowrate (µL/min)** | 600 | 600 | 600 | 600 | 600 | 600 |
| **%A** | 80 | 50 | 15 | 15 | 80 | 80 |
| **%B** | 20 | 50 | 85 | 85 | 20 | 20 |

### Peptide mapping

### - Tryptic digestion

Briefly, 10 µL of recombinant mAb (diluted at 1 mg/mL in 50 mM ammonium bicarbonate) were denatured and reduced by adding 1 µL of 1% ProteaseMax and 1 µL of 100 mM dithiothreitol followed by an incubation for 30 min at 56°C. Then, free cysteines were alkylated by adding 1 µL of 200 mM iodoacetamide prior to incubation for 45 min at RT in the dark. Then, digestion was performed by adding 35 µL of 50 mM ammonium bicarbonate and 1 µL of trypsin solution prepared at 1 µg/µL (overnight incubation at 37°C). Tryptic digestion was stopped by adding 1 µL of 100% formic acid. Finally, samples were centrifuged for 10 min at 13000 rpm and supernatants were transferred into LC-MS vial before analysis.

### - LC-MS/MS experiment

Tryptic digests were subjected to RP-HPLC-UV-ESI-MS and MS/MS analyses using a Vanquish Horizon UHPLC (Thermo Fisher Scientific) coupled to an Impact II mass spectrometer (Bruker Daltonics). Tryptic digests (1 µg) were loaded on an ACQUITY UPLC CSH C18 column (1.7 µm, 2.1x150 mm, Waters) thermostated at 60°C with a flow rate of 0.4 mL/min and eluted with the gradient presented in Table 3.

**Table 3: LC-MS gradient used for peptide mapping.**

| Buffer A: 0.1% FA; Buffer B: 0.1% FA in acetonitrile | | | | | | | | | |
|---|---|---|---|---|---|---|---|---|---|
| **Time (min)** | **0** | **3.0** | **8.0** | **13.0** | **40.0** | **40.1** | **45.0** | **45.1** | **50** |
| **%A** | 100 | 100 | 93 | 88 | 65 | 3 | 3 | 100 | 100 |
| **%B** | 0 | 0 | 7 | 12 | 35 | 97 | 97 | 0 | 0 |

MS analysis was performed using the Impact II mass spectrometer equipped with an ESI source (Bruker Daltonics). MS acquisition was performed in positive mode with end plate offset and capillary voltages set at 500 and 4500 V, respectively. Nebulizer and dry gas were set at 0.4 bar and 4.0 L/min (180°C), respectively. MS spectrum was acquired over the m/z range 50-2200 with a scan rate of 2 Hz. Acquisition was performed using the IDAS mode (Intensity Dependent Acquisition Speed) with a cycle time set to 2s. Calibration was carried out using the internal lockmass at 1221.9906. Peptide identification was performed using PEAKS XPro software (BSI informatics).

No incorporation of L-norvaline instead of Leucine was detected.

### Example 13: Small scale dynamic perfusion experiment

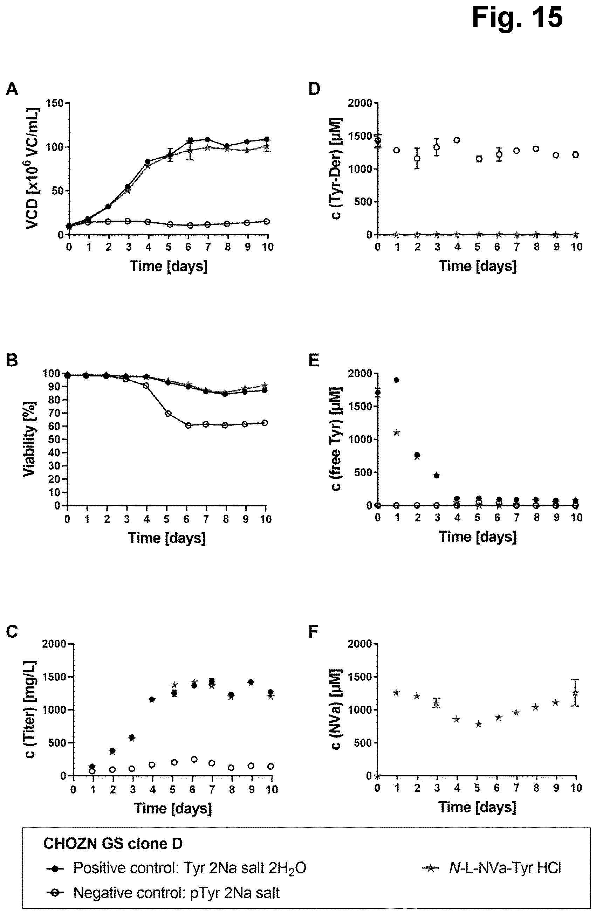

Figure 15 displays the cell performance of a CHOZN GS clone D, producing an IgG1 in a dynamic perfusion experiment. Overall results indicate a similar cell performance for *N*-L-NVa-Tyr HCl compared to the positive control Tyr 2Na salt 2H₂O (Figure 15A-C). Both conditions reached a maximum VCD in the range of 100 x10⁶ viable cells/mL on day 10. The positive control resulted in a maximum titer of 1425±39 mg/L and for *N*-L-NVa-Tyr HCl a titer of 1400±22 mg/L was reached after nine days in culture. Viability was constantly above 90 % until day five. From day six, viability dropped slightly and was maintained between 85 - 90 %.

The concentration of *N*-L-NVa-Tyr HCl of 1513 µM was fully depleted after one day in culture (Figure 15D), whereas the concentration of free Tyr increased to a maximum of 1106±52 µM on day one and afterwards decreased similarly to the positive control (Figure 15E). The NVa concentration was maintained around 1000 µM during the entire process. The negative control maintained a VCD at approximately 13 x10⁶ viable cells/mL, reached a maximum titer of 253±3 mg/L on day six, its initial concentration was maintained and no free Tyr was detected.

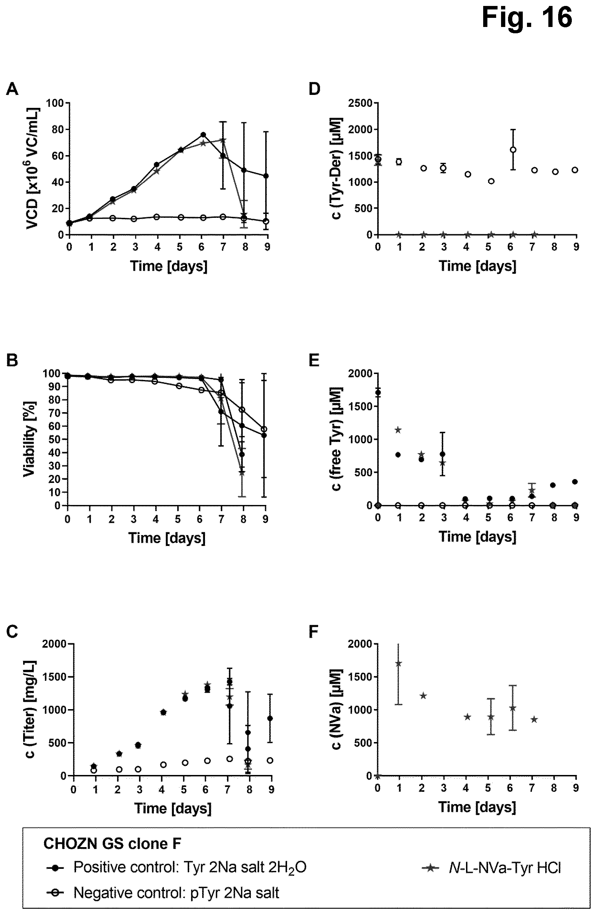

Figure 16 displays the cell performance of a CHOZN GS clone F, producing an IgG1 in a dynamic perfusion experiment. Overall results indicate a similar cell performance for *N*-L-NVa-Tyr HCl compared to the positive control Tyr 2Na salt 2H₂O (Figure 16A-C). Both conditions reached a maximum VCD in the range of 70 x10⁶ viable cells/mL on day six. The positive control resulted in a maximum titer of 1335±38 mg/L and for *N*-L-NVa-Tyr HCl a titer of 1379±23 mg/L was reached after nine days in culture. Viability was constantly above 95 % until day six. From day six viability dropped below 90 %.

The concentration of *N*-L-NVa-Tyr HCl of 1513 µM was fully depleted after one day in culture (Figure 16D, whereas the concentration of free Tyr increased to a maximum of 1143 µM on day one and afterwards decreased similar to the positive control (Figure 16E).

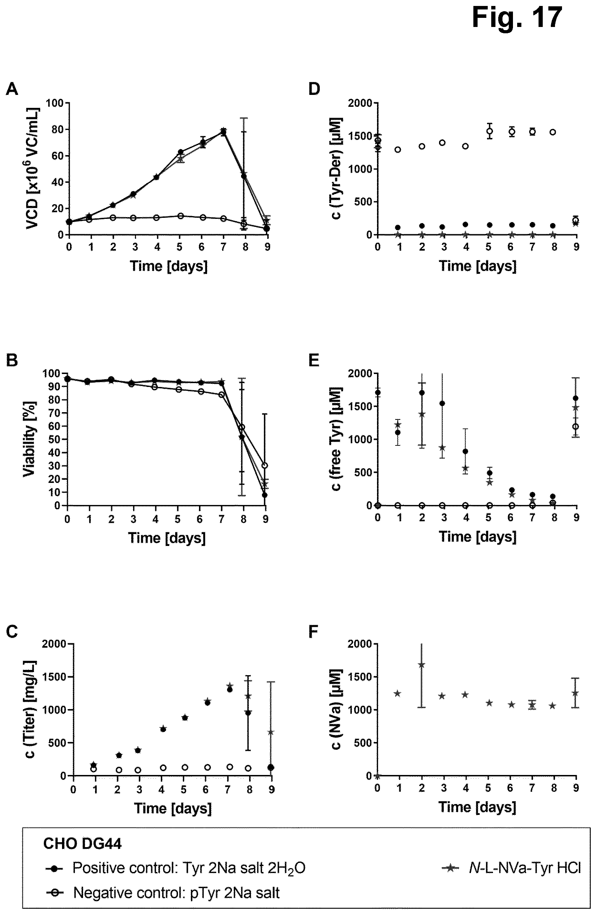

Figure 17 displays the cell performance of a CHO DG44, producing an IgG1 in a dynamic perfusion experiment. Overall results indicate a similar cell performance for *N*-L-NVa-Tyr HCl compared to the positive control Tyr 2Na salt 2H₂O (Figure 17A-C). Both conditions reached a maximum VCD in the range of 80 x10⁶ viable cells/mL on day six. The positive control resulted in a maximum titer of 1304±18 mg/L and for *N*-L-NVa-Tyr HCl a titer of 1360±3 mg/L was reached after seven days in culture. Viability was constantly above 90 % until day seven. Afterwards, viability dropped below 90 %.

The concentration of *N*-L-NVa-Tyr HCl of 1513 µM was fully depleted after one day in culture (Figure 17D), whereas the concentration of free Tyr increased to a maximum of 1384±470 µM on day two and afterwards decreased similarly to the positive control (Figure 17E).

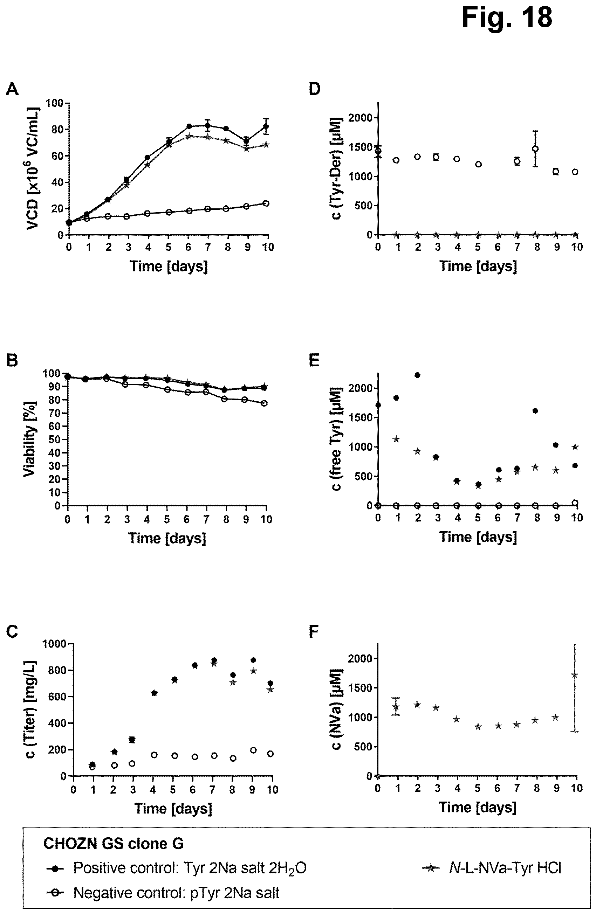

Figure 18 displays the cell performance of a CHOZN GS clone G, producing an IgG1 in a dynamic perfusion experiment. Overall results indicate a similar cell performance for *N*-L-NVa-Tyr HCl compared to the positive control Tyr 2Na salt 2H2O (Figure 18A-C). Both conditions reached a maximum VCD in the range of 100 x10⁶ viable cells/mL on day 10. The positive control resulted in a maximum titer of 1425±39 mg/L and for *N*-L-NVa-Tyr HCl a titer of 1400±22 mg/L was reached after nine days in culture. Viability was constantly above 90 % until day five. From day six, viability dropped slightly and maintained between 85 - 90 %.

The concentration of *N*-L-NVa-Tyr HCl of 1513 µM was fully depleted after one day in culture (Figure 18D), whereas the concentration of free Tyr increased to a maximum of 1131±169 µM on day one and afterwards decreased similarly to the positive control (Figure 18E).

## Claims

1. Cell culture medium comprising N-norvalyl-L-tyrosine.

2. Cell culture medium according to claim 1 **characterized in that** the cell culture medium comprises the hydrochloride salt of N-norvalyl-L-tyrosine.

3. Cell culture medium according to one or more of claims 1 to 2 **characterized in that** the medium comprises N-norvalyl-L-tyrosine but no tyrosine.

4. Cell culture medium according to claim 1 or claim 3 **characterized in that** the cell culture medium is a dry powder medium.

5. Cell culture medium according to one or more of claims 1 to 3 **characterized in that** the cell culture medium is a liquid medium having a pH of 8.5 or less and comprising N-norvalyl-L-tyrosine in a concentration between 10 mmol/L and 60 mmol/L.

6. Cell culture medium according to one or more of claims 1 to 5 **characterized in that** the concentration of the cell culture medium is in X-fold concentrated form relative to the concentration of said medium in use, whereby X is between 1.5 and 100.

7. Cell culture medium according to one or more of claims 1 to 6 **characterized in that** the pH of the liquid medium is between 6.0 and 8.5.

8. Cell culture medium according to one or more of claims 1 to 7 **characterized in that** the cell culture medium comprises at least one or more saccharide components, one or more amino acids, one or more vitamins or vitamin precursors, one or more salts, one or more buffer components, one or more co-factors and one or more nucleic acid components.

9. A method for producing a cell culture medium according to one or more of claims 1 to 8 by
a) mixing N-norvalyl-L-tyrosine with the other components of the cell culture medium
b) subjecting the mixture of step a) to milling

10. A method for producing a cell culture medium according to claim 9, **characterized in that** step b) is performed in a pin mill, fitz mill or a jet mill.

11. A method for producing a cell culture medium according to claim 9 or claim 10, **characterized in that** the mixture from step a) is cooled to a temperature below 0°C prior to milling.

12. A process for culturing cells by
a) providing a bioreactor
b) optionally generating a liquid cell culture medium by dissolving a dry powder medium according to one or more of claims 1 to 8 in an aqueous liquid
c) mixing the cells to be cultured with a liquid cell culture medium according to one or more of claims 1 to 8
d) incubating the mixture of step c).

13. Process of claim 12, **characterized in that** the bioreactor is a perfusion bioreactor.

14. Process according to claim 12 or claim 13, **characterized in that** the process for culturing cells is a process for perfusion cell culture, comprising culturing cells in a perfusion bioreactor with a media inlet and a harvest outlet whereby
i. continuously or one or several times during the cell culture process new liquid cell culture medium comprising N-norvalyl-L-tyrosine is inserted into the bioreactor via the media inlet
ii. continuously or one or several times during the cell culture process harvest is removed from the bioreactor via the harvest outlet

15. A process according to claim 14, **characterized in that** during perfusion phase cell culture medium comprising N-norvalyl-L-tyrosine is continuously inserted into the bioreactor via the media inlet and harvest is continuously removed from the bioreactor.

16. A fed batch process for culturing cells in a bioreactor by
a) Filling into a bioreactor cells and an aqueous cell culture medium
b) Incubating the cells in the bioreactor
c) Adding a cell culture medium, which is in this case a feed medium, to the bioreactor, continuously over the whole time or once or several times within the incubation of the cells in the bioreactor
whereby the feed medium is liquid a cell culture medium comprising N-norvalyl-L-tyrosine.

17. A fed batch process according to claim 16, **characterized in that** the liquid feed medium added in step c) has been prepared by dissolving a dry powder cell culture medium according to one or more of claims 1 to 8.

18. A fed batch process according to claim 16 or 17, **characterized in that** the liquid feed medium has a pH below pH 8.5 and comprises at least N-norvalyl-L-tyrosine in a concentration above 10 mmol/L.

19. A fed batch process according to one or more of claims 16 to 18, **characterized in that** the liquid feed medium does not comprise tyrosine.

## Patentansprüche

1. Zellkulturmedium enthaltend N-Norvalyl-L-tyrosin.

2. Zellkulturmedium nach Anspruch 1, **dadurch gekennzeichnet, dass** das Zellkulturmedium das Hydrochloridsalz von N-Norvalyl-L-tyrosin enthält.

3. Zellkulturmedium nach einem oder mehreren der Ansprüche 1 bis 2, **dadurch gekennzeichnet, dass** das Medium N-Norvalyl-L-tyrosin, aber kein Tyrosin enthält.

4. Zellkulturmedium nach Anspruch 1 oder Anspruch 3, **dadurch gekennzeichnet, dass** das Zellkulturmedium ein trockenes Pulvermedium ist.

5. Zellkulturmedium nach einem oder mehreren der Ansprüche 1 bis 3, **dadurch gekennzeichnet, dass** das Zellkulturmedium ein flüssiges Medium mit einem pH von 8,5 oder weniger und enthaltend N-Norvalyl-L-tyrosin in einer Konzentration zwischen 10 mmol/l und 60 mmol/l ist.

6. Zellkulturmedium nach einem oder mehreren der Ansprüche 1 bis 5, **dadurch gekennzeichnet, dass** die Konzentration des Zellkulturmediums in X-fach konzentrierter Form relativ zur Konzentration des Mediums bei der Verwendung ist, wobei X zwischen 1,5 und 100 liegt.

7. Zellkulturmedium nach einem oder mehreren der Ansprüche 1 bis 6, **dadurch gekennzeichnet, dass** der pH des flüssigen Mediums zwischen 6,0 und 8,5 liegt.

8. Zellkulturmedium nach einem oder mehreren der Ansprüche 1 bis 7, **dadurch gekennzeichnet, dass** das Zellkulturmedium mindestens eine oder mehrere Saccharidkomponenten, eine oder mehrere Aminosäuren, ein(e) oder mehrere Vitamine oder Vitaminvorstufen, ein oder mehrere Salze, eine oder mehrere Pufferkomponenten, einen oder mehrere Cofaktoren und eine oder mehrere Nukleinsäurekomponenten enthält.

9. Verfahren zur Herstellung eines Zellkulturmediums nach einem oder mehreren der Ansprüche 1 bis 8, indem man
a) N-Norvalyl-L-tyrosin mit den anderen Komponenten des Zellkulturmediums mischt,
b) die Mischung des Schritts a) einer Mahlung unterwirft.

10. Verfahren zur Herstellung eines Zellkulturmediums nach Anspruch 9, **dadurch gekennzeichnet, dass** der Schritt b) in einer Stiftmühle, Fitzmühle oder einer Strahlmühle durchgeführt wird.

11. Verfahren zur Herstellung eines Zellkulturmediums nach Anspruch 9 oder Anspruch 10, **dadurch gekennzeichnet, dass** die Mischung aus Schritt a) vor dem Mahlen auf eine Temperatur unter 0°C gekühlt wird.

12. Verfahren zur Kultivierung von Zellen, indem man
a) einen Bioreaktor bereitstellt,
b) gegebenenfalls ein flüssiges Zellkulturmedium erzeugt, indem man ein trockenes Pulvermedium nach einem oder mehreren der Ansprüche 1 bis 8 in einer wässrigen Flüssigkeit löst,
c) die zu kultivierenden Zellen mit einem flüssigen Zellkulturmedium nach einem oder mehreren der Ansprüche 1 bis 8 mischt,
d) die Mischung des Schritts c) inkubiert.

13. Verfahren des Anspruchs 12, **dadurch gekennzeichnet, dass** der Bioreaktor ein Perfusionsbioreaktor ist.

14. Verfahren nach Anspruch 12 oder Anspruch 13, **dadurch gekennzeichnet, dass** das Verfahren zur Kultivierung von Zellen ein Verfahren für die Perfusionszellkultur ist, umfassend die Kultivierung von Zellen in einem Perfusionsbioreaktor mit einem Medieneinlass und einem Ernteauslass, wobei
i. kontinuierlich oder einmal oder mehrmals während des Zellkulturverfahrens neues flüssiges Zellkulturmedium enthaltend N-Norvalyl-L-tyrosin über den Medieneinlass in den Bioreaktor eingebracht wird,
ii. kontinuierlich oder einmal oder mehrmals während des Zellkulturverfahrens Ernte über den Ernteauslass aus dem Bioreaktor entnommen wird.

15. Verfahren nach Anspruch 14, **dadurch gekennzeichnet, dass** während der Perfusionsphase kontinuierlich Zellkulturmedium enthaltend N-Norvalyl-L-tyrosin über den Medieneinlass in den Bioreaktor eingebracht wird und kontinuierlich Ernte aus dem Bioreaktor entnommen wird.

16. Fed-Batch-Verfahren zum Kultivieren von Zellen in einem Bioreaktor, indem man
a) einen Bioreaktor mit Zellen und einem wässrigen Zellkulturmedium befüllt,
b) die Zellen in dem Bioreaktor inkubiert,
c) ein Zellkulturmedium, das in diesem Fall ein Zufütterungsmedium ist, zum Bioreaktor hinzugibt, kontinuierlich über den ganzen Zeitraum oder einmal oder mehrmals innerhalb der Inkubation der Zellen in dem Bioreaktor,
wobei das Zufütterungsmedium ein flüssiges Zellkulturmedium enthaltend N-Norvalyl-L-tyrosin ist.

17. Fed-Batch-Verfahren nach Anspruch 16, **dadurch gekennzeichnet, dass** das in Schritt c) zugegebene flüssige Zufütterungsmedium hergestellt wurde, indem ein Trockenpulver-Zellkulturmedium nach einem oder mehreren der Ansprüche 1 bis 8 gelöst wurde.

18. Fed-Batch-Verfahren nach Anspruch 16 oder 17, **dadurch gekennzeichnet, dass** das flüssige Zufütterungsmedium einen pH unter pH 8,5 aufweist und mindestens N-Norvalyl-L-tyrosin in einer Konzentration über 10 mmol/l enthält.

19. Fed-Batch-Verfahren nach einem oder mehreren der Ansprüche 16 bis 18, **dadurch gekennzeichnet, dass** das flüssige Zufütterungsmedium kein Tyrosin enthält.

## Revendications

1. Milieu de culture cellulaire comprenant de la N-norvalyl-L-tyrosine.

2. Milieu de culture cellulaire selon la revendication 1, **caractérisé en ce que** le milieu de culture cellulaire comprend le sel de chlorhydrate de la N-norvalyl-L-tyrosine.

3. Milieu de culture cellulaire selon l'une ou plusieurs parmi les revendications 1 à 2, **caractérisé en ce que** le milieu comprend de la N-norvalyl-L-tyrosine mais pas de tyrosine.

4. Milieu de culture cellulaire selon la revendication 1 ou la revendication 3, **caractérisé en ce que** le milieu de culture cellulaire est un milieu en poudre sèche.

5. Milieu de culture cellulaire selon l'une ou plusieurs parmi les revendications 1 à 3, **caractérisé en ce que** le milieu de culture cellulaire est un milieu liquide ayant un pH de 8,5 ou moins et comprenant de la N-norvalyl-L-tyrosine selon une concentration comprise entre 10 mmol/L et 60 mmol/L.

6. Milieu de culture cellulaire selon l'une ou plusieurs parmi les revendications 1 à 5, **caractérisé en ce que** la concentration du milieu de culture cellulaire se présente sous une forme concentrée X fois par rapport à la concentration dudit milieu lors de son utilisation, où X est compris entre 1,5 et 100.

7. Milieu de culture cellulaire selon l'une ou plusieurs parmi les revendications 1 à 6, **caractérisé en ce que** le pH du milieu liquide est compris entre 6,0 et 8,5.

8. Milieu de culture cellulaire selon l'une ou plusieurs parmi les revendications 1 à 7, **caractérisé en ce que** le milieu de culture cellulaire comprend au moins un ou plusieurs composants saccharidiques, un ou plusieurs acides aminés, un(e) ou plusieurs vitamines ou précurseurs de vitamines, un ou plusieurs sels, un ou plusieurs composants de tampon, un ou plusieurs cofacteurs et un ou plusieurs composants d'acide nucléique.

9. Méthode de production d'un milieu de culture cellulaire selon l'une ou plusieurs parmi les revendications 1 à 8, par
a) le mélange de N-norvalyl-L-tyrosine avec les autres composants du milieu de culture cellulaire
b) la soumission du mélange de l'étape a) à un broyage.

10. Méthode de production d'un milieu de culture cellulaire selon la revendication 9, **caractérisée en ce que** l'étape b) est réalisée dans un broyeur à broches, un broyeur Fitz ou un broyeur à jet.

11. Méthode de production d'un milieu de culture cellulaire selon la revendication 9 ou la revendication 10, **caractérisée en ce que** le mélange de l'étape a) est refroidi jusqu'à une température inférieure à 0°C avant le broyage.

12. Procédé de culture de cellules, par
a) la mise à disposition d'un bioréacteur
b) éventuellement la génération d'un milieu de culture cellulaire liquide par la solubilisation d'un milieu en poudre sèche selon l'une ou plusieurs parmi les revendications 1 à 8 dans un liquide aqueux
c) le mélange des cellules à cultiver avec un milieu de culture cellulaire liquide selon l'une ou plusieurs parmi les revendications 1 à 8
d) l'incubation du mélange de l'étape c).

13. Procédé selon la revendication 12, **caractérisé en ce que** le bioréacteur est un bioréacteur à perfusion.

14. Procédé selon la revendication 12 ou la revendication 13, **caractérisé en ce que** le procédé de culture de cellules est un procédé de culture cellulaire par perfusion, comprenant la culture de cellules dans un bioréacteur par perfusion muni d'une entrée de milieu et d'une sortie de récolte, dans lequel
i. en continu ou une ou plusieurs fois au cours du procédé de culture cellulaire, un nouveau milieu de culture cellulaire liquide comprenant de la N-norvalyl-L-tyrosine est introduit dans le bioréacteur via l'entrée de milieu
ii. en continu ou une ou plusieurs fois au cours du procédé de culture cellulaire, la récolte est récupérée à partir du bioréacteur via la sortie de récolte.

15. Procédé selon la revendication 14, **caractérisé en ce que**, lors de la phase de perfusion, un milieu de culture cellulaire comprenant de la N-norvalyl-L-tyrosine est introduit en continu dans le bioréacteur via l'entrée de milieu et la récolte est récupérée en continu à partir du bioréacteur.

16. Procédé de culture de cellules par alimentation discontinue dans un bioréacteur, par
a) l'introduction, dans un bioréacteur, de cellules et d'un milieu aqueux de culture cellulaire
b) l'incubation des cellules dans le bioréacteur
c) l'addition d'un milieu de culture cellulaire, en l'occurrence un milieu d'alimentation, au bioréacteur, en continu sur toute la durée ou une fois ou plusieurs fois au cours de l'incubation des cellules dans le bioréacteur
dans lequel le milieu d'alimentation est un milieu de culture cellulaire liquide comprenant de la N-norvalyl-L-tyrosine.

17. Procédé par alimentation discontinue selon la revendication 16, **caractérisé en ce que** le milieu d'alimentation liquide ajouté dans l'étape c) a été préparé par la solubilisation d'un milieu en poudre sèche de culture cellulaire selon l'une ou plusieurs parmi les revendications 1 à 8.

18. Procédé par alimentation discontinue selon la revendication 16 ou 17, **caractérisé en ce que** le milieu d'alimentation liquide présente un pH inférieur à pH 8,5 et comprend au moins de la N-norvalyl-L-tyrosine selon une concentration supérieure à 10 mmol/L.

19. Procédé par alimentation discontinue selon l'une ou plusieurs parmi les revendications 16 à 18, **caractérisé en ce que** le milieu d'alimentation liquide ne comprend pas de tyrosine.
